# EUROPEAN PATENT APPLICATION

(11) **EP 1 566 449 A1**
(43) Date of publication of application: **24.08.2005**
(21) Application number: 04003654.3
(22) Date of filing: 18.02.2004
(51) Int. Cl.: C12Q 1/68

(54) **Use of haplotypes and SNPs in lipid-relevant genes for the analyses and diagnosis of cardiovascular diseases**

(71) Applicant: MAX-DELBRÜCK-CENTRUM FÜR MOLEKULARE MEDIZIN, 13092 Berlin (DE)
(72) Inventor: Nürnberg, Peter, 10117 Berlin (DE); Reich, Jens-Georg, 16278 Angermünde (DE); Luft, Friedrich, 16341 Schwanbeck (DE); Knoblauch, Hans, 10967 Berlin (DE); Bauerfeind, Anja, 10437 Berlin (DE); Luganskaja, Tatjana, 12309 Berlin (DE); Rohde, Klaus, 13125 Berlin (DE)
(74) Representative: Krauss, Jan

(57) **Abstract**

The present invention relates to an *in vitro* method for diagnosing a genetic predisposition or susceptibility for a cardiovascular disease, condition or disorder in a mammal which comprises detecting of at least three particular single nucleotide polymorphisms (SNP) in a sample obtained from said mammal in at least one of a genomic locus-derived nucleic acid or fragment thereof of the loci APOB, APOE, ABCA1, CETP, LCAT, LIPC, LPL, LDLR, APOC2, APOA5, APOA4, APOC3, and APOA1. Furthermore, the present invention relates to the improved diagnosis that is based on the analysis of several haplotypes for the above-mentioned loci (i.e. a combination of said haplotypes).

## Description

The present invention relates to an *in vitro* method for diagnosing a genetic predisposition or susceptibility for a cardiovascular disease, condition or disorder in a mammal which comprises detecting of at least three particular single nucleotide polymorphisms (SNP) in a sample obtained from said mammal in at least one of a genomic locus-derived nucleic acid or fragment thereof of the loci APOB, APOE, ABCA1, CETP, LCAT, LIPC, LPL, LDLR, APOC2, APOA5, APOA4, APOC3, and APOA1. Furthermore, the present invention relates to the improved diagnosis that is based on the analysis of several haplotypes for the above-mentioned loci (i.e. a combination of said haplotypes).

### BACKGROUND OF THE INVENTION

Cardiovascular disease (CVD) is the leading cause of death world-wide and serum low-density cholesterol (LDL) and high-density cholesterol (HDL) are primary risk factors (Murray, C. J. L, and Lopez, A. D. (1997) Mortality by cause for eight regions of the world: Global burden of disease study. *Lancet,* **349,** 1269-1276). Major mutations have been described in genes coding for the low-density lipoprotein cholesterol receptor, apolipoprotein B, and more recently, LDLR adapter protein (Umans-Eckenhausen, M.A., Defesche, J C., Sijbrands, E. J., Scheerder, R. L., and Kastelein, J. J. (2001) Review of first 5 years of screening for familial hypercholesterolaemia in the Netherlands. *Lancet,* **357**,165-168; Garcia, C. K., Wilund, K., Arca, M., Zuliani, G., Fellin, R., Maioli, M., Calandra, S., Bertolini, S., Cossu, F., Grishin, N., et al. (2001) Autosomal recessive hypercholesterolemia caused by mutations in a putative LDL receptor adaptor protein. *Science,* **292,** 1394-1398). These Mendelian conditions are mechanistically highly informative, but are relatively infrequent or rare. In the general population, SNP variants in known genes important to lipid metabolism probably account for most of the genetic variance. A few candidate genes which contribute to this genetic variability as e.g. the Apolipoprotein E gene have been identified, but in general the picture of all genetic factors is far from being complete. The present inventors recently modelled the interactions of six genes important to lipid metabolism mathematically by means of differential equations (Knoblauch, H., Schuster, H., Luft, F. C., and Reich J. (2000) A pathway model of lipid metabolism to predict the effect of genetic variability on lipid levels. *J. Mol. Med.,* **78**, 507-515). The present inventors then selected SNPs in these genes, namely cholesteryl ester transfer protein *(CETP),* lipoprotein lipase (*LPL*)*,* hepatic triglyceride lipase (*LIPC*), low density lipoprotein cholesterol receptor (*LDLR*), apolipoprotein E (*APOE*), and lecithin:cholesterol acyltransferase (*LCAT*) genes and studied 625 individuals from 186 German families (Knoblauch H, Bauerfeind A, Krahenbuhl C, Daury A, Rohde K, Bejanin S, Essioux L, Schuster H, Luft F. C, Reich JG. (2002) Common haplotypes in five genes influence genetic variance of LDL and HDL cholesterol in the general population. *Hum. Mol. Genet.* **11**, 1477-1485). The present inventors found that allelic association of haplotypes was highly significant for HDL, LDL, and the LDL/HDL ratio. Haplotypes derived from SNP combinations resulted in greater significance than did single SNPs in the genotype-phenotype association analysis. In recent studies the heritability of lipid phenotypes is around 50% (Higgins, M. (2000). Epidemiology and prevention of coronary heart disease in families. *Am. J. Med.,* **108**, 387-395; Williams R. R., Hunt S. C., Hopkins P. N., Hasstedt S. J., Berry T. D., Barlow G. K., Stults B. M., Schumacher M. C., Ludwig E. H., et al. (1993) Genetic basis of familial dyslipidemia and hypertension: 15-year results from Utah. *Am. J Hypertens.* **6**, 319S-327S). Based on lower heritability estimates (33% for LDL and 45% for HDL 45%) from the present previous study the present inventors estimated that about 58%and 47% of the genetic variance for LDL and HDL, respectively could be "predicted" by haplotypes.

High levels of low density lipoprotein (LDL-) cholesterol, low levels of high density lipoprotein (HDL)-cholesterol, and to a lesser extent elevated plasma triglyceride are also important risk factors for arteriosclerosis and subsequent diseases, e.g. cardiovascular disease, myocardial infarction, cerebrovascular disease, stroke, dementia, thrombosis, pulmonary embolism and renal infarction.

In general, an analysis of complex genetic traits presents several difficulties. The majority of synonymous changes or intronic SNPs will have no physiological impact. Nevertheless, these SNPs may serve as linkage disequilibrium (LD) markers. Strong LD between SNPs may be anticipated in the present candidate genes, since they typically extend over genomic regions not larger than 30 kbp (Daley M. J., Rioux J. D., Schaffner S, Hudson T. J., Lander E. S. (2001) High-resolution haplotype structure in the human genome. *Nat. Genet.* **29:** 229-232; Patil N, Bemo A. J., Hinds D. A., Barrett W. A., Doshi J. M., Hacker C. R., Kautzer C. R., Lee D. H., Marjoribanks C, McDonough D. P., et al. (2001) Blocks of limited haplotype diversity revealed by high-resolution scanning of human chromosome 21. *Science* **294,** 1719-1723; Reich D. E., Cargill M, Bolk S, Ireland J, Sabeti P. C., Richter D. J., Lavery T, Kouyoumjian R, Farhadian S. F., Ward R. et al. (2001) Linkage disequilibrium in the human genome. *Nature,* **411,** 199-204; Abecasis G. R., Noguchi E, Heinzmann A, Traherne J. A., Bhattacharyya S, Leaves N. I., Anderson G. G., Zhang Y, Lench N. J., Carey A, et al. (2001) Extent and distribution of linkage disequilibrium in three genomic regions. *Am. J. Hum. Genet,* **68:** 191-197; Jeffreys A. J., Ritchie A, Neumann R. (2000) High resolution analysis of haplotype diversity and meiotic crossover in the human TAP2 recombination hotspot. *Hum. Mol. Genet.* **22**, 725-733; Gabriel S. B., Schaffner S, Nguyen H, Moore J. M., Roy J, Blumenstiel B, Higgins J, Defeleice M, Lochner A, Faggart M, et al. (2002) The structure of haplotype blocks in the human genome. *Science* **296,** 2225-2229; and Phillips M. W. S., Lawrence R, Sachidanandam R, Cardon L. R. et al. (2003) Chromosome-wide distribution of haplotype blocks and the role of recombination hot spots. Nat. Genet. 33, 382-387).

Furthermore, the LD profile along chromosomes does not decrease linearly with the physical distance. The population-genetic process of mutation and recombination has considerably stochastic scatter; population bottlenecks may complicate the picture. The present inventors have relied on a variance component model, since simple regression analysis is not valid in situations where phenotypes are correlated between family members (Falconer D. S. and MacKay T. F. C. (1996) An introduction into quantitative genetics. 4^{th} ed., Longman, Harlow (GB); Amos, C. I. (1994) Robust Variance-Components Approach for Assessing Genetic Linkage in Pedigrees. *Am. J. Hum. Genet.* **54,** 535-543; Fulker, D. W., Cherny, S. S., Sham, P. C., and Hewitt, J. K. (1999) Combined linkage and association analysis for quantitative traits. *Am. J. Hum. Genet.* **64,** 259-267; Sham, P. C., Cherny, S. S., Purcell, S. and Hewitt, J. K. (2000) Power of linkage versus association analysis of quantitative traits, by use of variance components models, for sibship data. *Am. J. Hum. Genet.,* **66,** 1616-1630; Abecasis, G. R., Cookson, W. O. C., and Cardon, L. R. (2000) Pedigree tests of transmission equilibrium. *Eur. J. Hum. Genet.,* **8,** 545-551; and Abecasis, G. R., Cardon, L. R., and Cookson, W. O. (2000) A general test of association for quantitative traits in nuclear families. *Am. J. Hum. Genet.,* **66,** 279-292.). Moreover, each individual locus may contribute only about 5 to 10% to the total variation. For example, *APOE* that has a large effect accounts for less than 10% of the total cholesterol variance (Sing C. F. & Davignon J. (1985) The role of apolipoprotein E polymorphism in determining normal plasma lipid and lipoprotein variation. *Am. J. Hum. Genet.* 37,268-285; Boerwinkle E, Visvikis S, Welsh D, Steinmetz J, Hanash S. M., Sing C. F. (1997) Use of Measured Genotype Information in the Analysis of Quantitative Phenotypes in Man. *Amer. J. Med. Genet.,* **27**, 567-582).

Sing and Davignon (Sing C. F. & Davignon J. (1985) The role of apolipoprotein E polymorphism in determining normal plasma lipid and lipoprotein variation. *Am. J. Hum. Genet.* 37,268-285) reported that *APOE* accounted for about 10% of the LDL phenotypic variance, which is very much in accord with the present findings (polygenic variance of LDL is 26%; 67% of this variance is due to genes analysed; 50% are contributed by APOE).

Morabia et al. (Morabia, A., Cayanis, E., Costanza, M. C., Ross, B. M., Flherty, M. S., Alvin, G. B., Das, K., Conrad Gilliam, T. (2003) Association of extreme blood lipid profile phenotypic variation with 11 reverse cholesterol transport genes and 10 non-genetic cardiovascular disease risk factors. *Hum. Mol. Genet.* **12**, 2733-2743) recently reported on extreme blood-lipid profiles in a cohort of patients who underwent genotyping in 11 lipid metabolism-relevant genes. They relied on a population-based sample (as did the present inventors) and had at their disposal numerous demographic risk parameters. In their association study, they classified 185 subjects as "cases" who were in the upper third of LDL and lower third of HDL values (worst-possible LDL/HDL ratio). They tested numerous SNPs and genes that were also examined in the present invention. The entire sample comprised 1708 persons. They then performed a logistic regression analysis and showed that a subset of 9 genetic variants in 7 candidate genes, coupled with 5 environmental risk factors, produced a model that may be relevant for future risk assessment.

Despite the recent improvements in the genetic analysis and/or "prediction" of CVD, still improved methods are needed in order to expand and improve the reliability of said methods in order to fully exploit the diagnostic potential of the LD-profile of a particular patient and/or subject.

Accordingly, the object of the present invention, in a first embodiment of the present invention, is achieved by *an in vitro* method for diagnosing a genetic predisposition or susceptibility for a cardiovascular disease, condition or disorder in a mammal comprising detecting in a sample obtained from said mammal at least three single nucleotide polymorphisms (SNPs) in a nucleic acid or fragment thereof wherein said nucleic acid or fragment thereof is derived from at least one of a genomic locus of the group consisting of APOB, APOE, ABCA1, CETP, LCAT, LIPC, LPL, LDLR, APOC2, APOA5, APOA4, APOC3, and APOA1, and wherein said at least three SNPs are selected from the group consisting of the SNPs rs512535, rs1367117, rs520354, rs679899, rs673548, rs693, rs1800479, rs1801703, and rs1042034 of APOB, rs449647, rs405509, rs440446, rs769450, rs429358, and rs7412 of APOE, rs2422493, rs1800977, rs2575879, rs1800978, rs1999429, rs2230806, rs2274873, rs4149313, rs2066716, and rs363717 of ABCA1, rs4783961, rs1800776, rs1800775, rs711752, rs708272, rs158478, rs1532625, rs289718, rs289719, rs5880, rs5882, and rs1801706 of CETP, rs1109166, rs4986970, and rs5923 of LCAT, rs723967, rs1800588, rs1869144, rs6078, rs690, rs6082, rs6083, rs6084, and rs6074 of LIPC, rs1800590, rs1031045, rs253, rs268, rs269, rs2075651, rs270, rs285, rs320, rs328, and rs3289 of LPL, rs2228671, rs885765, rs5930, rs5925, rs5927, and rs1433099 of LDLR, rs5167, rs2288911, rs5120, and rs892101 of APOC2, rs2266788, rs2072560, rs3135506, rs662799, rs2266789, and rs1729408 of APOA5, rs675, rs5104, rs5092, and rs2542051 of APOA4, rs2854116, rs4520, and rs5128 of APOC3, and rs525028, rs5081rs5070, rs1799837, and rs5069 of APOA1. By the analysis of said three SNPs, at least one allele-specific haplotype is determined for at least one of the above-mentioned genes.

The above-described difficulties in the present methods for the diagnosis of CVD caused the present inventors to expand the sample size of the present earlier study as the present inventors incorporated additional genes. The important finding in the present invention was that the present inventors were able to explain a major part of the genetic variance for LDL, HDL, and especially LDL/HDL ratio in the general German population on the basis of the genetic variants identified in (up to) 13 candidate genes important to lipid metabolism. The normal population is highly relevant in terms of investigation since more than half of "normal" people can expect to die of cardiovascular disease (CVD). The levels of LDL, HDL and LDL/HDL ratio are important clinical indicators of the cardiovascular risk or degree of protection for each individual. Under controlled standardised physiological conditions, these levels do not vary much in any given individual. The variation between individuals is larger. The variation between members of an out-bred human population is heritable to a degree of about 50% (Higgins, M. (2000). Epidemiology and prevention of coronary heart disease in families. *Am. J. Med.,* **108,** 387-395; Williams R. R., Hunt S. C., Hopkins P. N., Hasstedt S. J., Berry T. D., Barlow G. K., Stults B. M., Schumacher M. C., Ludwig E. H., et al. (1993) Genetic basis of familial dyslipidemia and hypertension: 15-year results from Utah. *Am. J. Hypertens.* **6**, 319S-327S; Jeffreys A. J., Ritchie A, Neumann R. (2000) High resolution analysis of haplotype diversity and meiotic crossover in the human TAP2 recombination hotspot. *Hum. Mol. Genet..* **22,** 725-733).

In the context of the present invention, the term "arteriosclerosis involving conditions" includes conditions that include the genetic predisposition or susceptibility for a cardiovascular disease, condition, or disorder that can involve myocardial infarction, stroke, and/or abnormal blood lipid levels. Further arteriosclerosis involving conditions are in general, cardiovascular disease, myocardial infarction, stroke, cerebrovascular disease, dementia, thrombosis, pulmonary embolism, renal infarction as well as further disorders associated with altered lipid levels, such as levels of LDL, HDL, and especially the LDL/HDL ratio. Finally, also forms of conditions like overweight and obesity are included in this term as well.

"Predisposed" or "susceptible" to arteriosclerosis involving conditions in the context of the present invention shall mean that the base configuration of the individual allows to differentiate individuals which develop, or have developed an elevated total cholesterol level, an elevated LDL cholesterol level, a reduced HDL cholesterol level, or elevated triglyceride levels or a combination of the above. Furthermore, "predisposed to cardiovascular disease means" that the base configuration of the individual allows to differentiate individuals which are more likely to suffer from myocardial infarction or which are less likely to survive a myocardial infarction event.

"Protected from arteriosclerosis involving conditions " in the context of the present invention shall mean that the base configuration of the individual allows to differentiate individuals which develop, or have developed a reduced total cholesterol level, a reduced LDL cholesterol level, an elevated HDL cholesterol level, a shift of the LDL/HDL ratio or reduced triglyceride levels or a combination of the above. Furthermore, protected from arteriosclerosis involving conditions shall mean that the base configuration of the individual allows to differentiate individuals which are less likely to suffer from myocardial infarction or which are more likely to survive a myocardial infarction event.

In the context of the present invention, a "gene" means any one of the genetic-loci from the group consisting of the areas the encode for APOB, APOE, ABCA1, CETP, LCAT, LIPC, LPL, LDLR, APOC2, APOA5, APOA4, APOC3 or APOA1. These loci encompass the region of the promoter of said particular coding region, the introns, the exons, intergenic regions and 3' and/or 5'-untranscribed regions (3' and/or 5'-UTRs). In particular, the regions of the SNPs as described in table 1 below are regarded as belonging to "genes" of the present invention.

According to another aspect of the present invention, the inventive method allows for the identification of individuals that are protected from said arteriosclerosis involving conditions, i.e. are less likely to suffer from said arteriosclerosis involving conditions. Preferably, said genetic predisposition or susceptibility for an arteriosclerosis involving conditions involves myocardial infarction, stroke, and/or abnormal blood lipid levels. Normally, the method is performed on mammals, e.g. humans.

"Detecting a nucleotide alteration" in the context of the present invention shall mean that at least the base sequence of one diagnostically relevant genetic position as provided for by the present invention is determined. It is of course possible to do this by determining the base on a) the plus strand of genomic DNA or b) the minus strand of genomic DNA. Of course, in other embodiments of this invention the sequence of nucleotides upstream or downstream of the analysed position are also determined. The method according to the invention is not limited to determining exactly the nucleotides shown therein. It is only important to determine the variant site therein.

Although the method of the invention can be performed in mammals in general, it is preferred to perform the method with human samples. Such samples may be, e.g. blood, urine, semen, hair or any other tissue containing at least the nucleic acid to be analysed.

According to the method of the present invention, alleles of the genes APOB, APOE, ABCA1, CETP, LCAT, LIPC, LPL, LDLR, APOC2, APOA5, APOA4, APOC3, and APOA1 are detected. Allelic variants of the genes encompass all forms of polymorphisms or mutations including deletions, insertions and point mutations at positions of the sequences of said genes. Polymorphic alleles or point mutations may result in altered gene function. Individual alleles of the polymorphisms described herein can be combined with one another or with other neighbouring alleles of polymorphisms in the form of a diagnostic chromosome specific allele combination, i.e. a haplotype. Such a haplotype exhibits a greater diagnostic power than the individual polymorphism as such. In addition, several haplotypes of the present invention can be combined to exhibit even greater diagnostic power (i.e. statistical relevance) with respect to a particular "CVD-phenotype" of a patient under analysis.

Applicable diagnostic techniques include, but are not limited to, DNA sequencing including mini-sequencing, primer extension, hybridisation with allele-specific oligonucleotides (ASO), oligonucleotide ligation assays (OLA), PCR using allele-specific primers (ARMS), dot blot analysis, flap probe cleavage approaches, restriction fragment length polymorphism (RFLP), kinetic PCR, and PCR-SSCP, fluorescent in situ hybridisation (FISH), pulsed field gel electrophoresis (PFGE) analysis, Southern blot analysis, single stranded conformation analysis (SSCA), denaturing gradient gel electrophoresis (DGGE), temperature gradient gel electrophoresis (TGGE), denaturing HPLC (DHPLC), and RNAse protection assays, all of which are presently known to the person skilled in the art and discussed in detail further below.

The nucleic acid that forms part of the method according to the present invention can be DNA, genomic DNA, RNA, cDNA, hnRNA and/or mRNA. The detection can be accomplished by sequencing, mini-sequencing, hybridisation, restriction fragment analysis, oligonucleotide ligation assay or allele specific PCR.

The presence of the disease predisposition due to germline polymorphisms/variations of the genes APOB, APOE, ABCA1, CETP, LCAT, LIPC, LPL, LDLR, APOC2, APOA5, APOA4, APOC3, and/or APOA1 at the altered sites can be ascertained by testing any tissue of the human for those variations. For instance, a person who has inherited specific pattern of polymorphisms in the genes, will carry a higher risk of developing metabolic derailment or be prone to develop acute disease. The presence of such a polymorphism/mutation can be determined by extracting DNA from any tissue of the body. For example, blood can be drawn and DNA extracted from blood cells and analysed. Moreover, prenatal diagnosis of the disease will be possible by testing foetal cells, placental cells or amniotic cells for mutations in the gene. There are several methods that allow the detection of specific alleles, and some of these methods are discussed here:

For a known polymorphism, direct determination of the respective genotype is usually the method of choice. State of the art approaches for industrial high-throughput genotyping today rely on one of four different mechanisms: allele-specific primer extension, allele-specific hybridization, allele-specific oligonucleotide ligation and allele-specific cleavage of a flap probe (Kwok, *Pharmacogenomics* 1,95(2000)). Sequencing or mini-sequencing protocols are part of the primer extension methods, e.g. genomic DNA sequencing, either manual or by automated means can detect sequence variations of the genes APOB, APOE, ABCA1, CETP, LCAT, LIPC, LPL, LDLR, APOC2, APOA5, APOA4, APOC3, and APOA1 *(Nucleic Acids Res* 25,2032-2034(1997)). Minisequencing (primer extension) technology is based on determining the sequence at a specific base by allowing the elongation of a primer by one base directly at the variant site (Landegren et al., *Genome Res.* **8:** 769-76 (1998)). Short sequence reactions coupled with an alternative detection method are the nature of real time pyrophosphate sequencing (Nyren et al., *Science* 281:363 (1998)).

Allele-specific hybridisation protocols rely on probes detecting one or several of the alleles present at the SNP positions. Several techniques were developed for detection of an hybridisation event. In the 5' nuclease assay and in the molecular beacon assay the hybridization probes are fluorescently labelled and probe binding is detected via changes in the behavior of the fluorescent label (Livak, *Genet. Anal.* 14, 143 (1999); Tyagi et al., *Nat. Biotechnol.* 16, 49 (1998)). Hybridisation events may occur in liquid phase or with either the probe or the target bound to a solid surface. Hybridisation is thus also used when arrays (microchips) are used for genotyping purposes. This technique of nucleic acid analysis is also applicable to the present invention. A chip typically consists of thousands of distinct nucleotide probes which are built up in an array on a silicon chip. Nucleic acid to be analysed is fluorescently labelled (in the present case a sample containing the genes APOB, APOE, ABCA1, CETP, LCAT, LIPC, LPL, LDLR, APOC2, APOA5, APOA4, APOC3, and APOA1 or a portion thereof spanning the alterations) and hybridised to the probes on the chip. This method is one of parallel processing of thousands of probes at once and can tremendously accelerate the analysis. In several publications the use of this method is described (Hacia et al., *Nature Genetics* 14, 441 (1996); Shoemaker et al., *Nature Genetics* 14, 450 (1996); Chee et al., *Science* 274, 610 (1996); De-Risi et al., *Nature Genetics* 14, 457 (1996), Fan et al., *Genome Res,* 10, 853 (2000)).

Allele-specific oligonucleotide ligation assays have a high specificity. Oligonucleotides differing in the allele-specific base at the 5'- or 3'-end are only processed by a ligation reaction if they are perfectly bound to the template at the respective oligonucleotide end. This method has been coupled with fluorescence resonance energy transfer (FRET) labelling to create a homogeneous assay system (Chen et al. *Genome Res.* 8, 549 (1998)). Allele-specific cleavage of a flap probe use the property of recently discovered flap endonucleases (cleavases) to cleave structures created by two overlapping oligonucleotides. In this approach two overlapping oligonucleotides are bound to the polymorphic site. Which oligo has had a perfect match to the target sequence is then detected by the cleavage reaction (Lyamichev et al., *Nat. Biotechnol.* 17:292 (1999)).

Other methods which detect specific base variations usually allow a lower throughput only such as the allele-specific oligonucleotide (ASO) hybridisation. For allele-specific PCR, primers are used which hybridise at their 3' ends to one of the particular APOB, APOE, ABCA1, CETP, LCAT, LIPC, LPL, LDLR, APOC2, APOA5, APOA4, APOC3, and/or APOA1 base variations according to the invention. Only for alleles which are present, a respective PCR product is observed (Ruano and Kidd, *Nucleic Acids Res* 17, 8392 (1989)). A specificity increasing modification of allele-specific PCR is the Amplification Refractory Mutation System, as disclosed in European Patent Application Publication No. 0332435 and in Newton et al., *Nucleic Acids Res* 17, 2503 (1989). If the variations lead to changes in the specific recognition sites of nucleic acid processing enzymes methodologies such as restriction fragment length polymorphism (RFLP) probes or PCR-RFLP methods may also be used to detect these variations.

Other approaches detect only that changes with respect to a reference sequence are present in a nucleic acid. Many of these methods are based on the formation of mismatches when both SNP variants are present in the same sample. A currently very popular method is the use of denaturing high performance liquid chromatography to separate heteroduplex from homoduplex molecules (DHPLC; Oefner, P.J. et al. *Am J Hum Genet* 57 (Suppl.), A266 (1995)). Another method is denaturing gradient gel electrophoresis (DGGE) (Wartell et al., *Nucleic Acids Res* 18, 2699, (1990); Sheffield et al., *Proc Natl Acad Sci USA* 86, 232 (1989)). By using DGGE, variations in the DNA can be detected by differential migration rates of allelic variants in a denaturing gradient gel. A variation is the clamped denaturing gel electrophoresis (CDGE; Sheffield et al., *Am J Hum Genet* 49, 699 (1991)), heteroduplex analysis (HA; White et al., *Genomics* 4, 560 (1992)) and chemical mismatch cleavage (CMC; Grompe et al. *Proc Natl Acad Sci USA* 86, 5888 (1989)). The use of proteins which recognise nucleotide mismatches, such as the *E. coli* mutS protein may help in detecting mismatched DNA molecules (Modrich, *Ann. Rev. Genetics,* 25, 229 (1991)). In the mutS assay, the protein binds only to sequences that contain a nucleotide mismatch in a heteroduplex between mutant and wild-type sequences. RNase protection assays are another option (Finkelstein et al., *Genomics 7,* 167 (1990)). The RNAse protection assay involves cleavage of the mutant fragment into two or more smaller fragments. Another way is to make use of the single-stranded conformation polymorphism assay (SSCP; Orita et al., *Proc Natl Acad Sci USA* 86, 2766 (1989)). Variations in the DNA sequence of the genes APOB, APOE, ABCA1, CETP, LCAT, LIPC, LPL, LDLR, APOC2, APOA5, APOA4, APOC3, and/or APOA1 from the reference sequences will be detected due to a shifted mobility of the corresponding DNA-fragments in SSCP gels. SSCP detects bands which migrate differently because the variation causes a difference in single strand, intra-molecular base pairing. Other methods detect specific types of mutations such as deletions, duplications or insertions, for instance a protein truncation assay or the asymmetric assay. These assays however, will not detect missense mutations.

Indirect methods as described above for the detection of sequence variations would be particularly useful for screening relatives for the presence of a sequence variation found previously in an affected family member.

Other approaches for detecting small sequence variations as known for those skilled in the art can be used.

Detection of polymorphisms/point mutations may be accomplished by amplification, for instance by PCR, from genomic or cDNA and sequencing of the amplified nucleic or by molecular cloning of the APOB, APOE, ABCA1, CETP, LCAT, LIPC, LPL, LDLR, APOC2, APOA5, APOA4, APOC3 and/or APOA1 allele and sequencing the allele using techniques well known in the art.

In a preferred embodiment of the present invention at least three of the nucleotide alterations of at least one of the genes APOB, APOE, ABCA1, CETP, LCAT, LIPC, LPL, LDLR, APOC2, APOA5, APOA4, APOC3 and/or APOA1 are detected in a sample by hybridising a gene probe which specifically hybridises to the alternative forms of the polymorphism/variant nucleic acids containing at least one of said alterations of the genes from said mammalian sample and detecting the presence of a hybridisation product, wherein the presence of said product indicates the presence of said base configuration in said sample.

Herein, the gene probes are e.g. oligomeric DNA sequences of 15 to 50 bases which are synthesised with at least one variant base, preferentially both variant bases and hybridised individually under stringent conditions allowing single base variant discrimination. Alternatively, under less stringent conditions a set of gene probes of 15 to 50 bases in length representing all four potential bases at the polymorphic position of the analysed DNA strand can be used for typing by hybridisation. In this case results from all hybridisation experiments with differing degrees of base complementarity need to be analysed by an algorithm to predict the final nucleotide configuration at the variant site.

In a preferred embodiment of the invention a nucleotide alteration is detected by amplifying all or part of the genes APOB, APOE, ABCA1, CETP, LCAT, LIPC, LPL, LDLR, APOC2, APOA5, APOA4, APOC3, and/or APOA1 in said sample using a set of, e.g. conserved primers specific for the gene to produce amplified nucleic acids, sequencing the amplified nucleic acids and detecting the presence of the nucleotide alterations set forth in Table 1. Examples of primers which amplify the region around the polymorphism can be easily designed by the person of skill in the art. Preferably, a combination of haplotypes of the loci APOB, APOE, ABCA1, CETP, LCAT, LIPC, LPL, LDLR, APOC2, APOA5, APOA4, APOC3, and APOA1 is detected, consisting of between 2 to 13 SNP haplotypes according to the present invention. Other examples are 3, 4, 5, 6, 7, 8, 9, 10, 11 or 12 SNP haplotypes. All relevant haplotypes can be found in the tables as attached herein below. Preferred combinations of haplotypes to be analysed for can be (see table 2 for numbers and abbreviations): APOB, 1 - 4 hap 01 and/or hap 07 (28.0% Increase LDL, p=0.002) and/or 5 - 9 hap 05 (19.8% LDL lowering effect, p=0.002) and/or APOE, hap 04 and/or hap 09 and/or ABCA1 1, 3 - 6 hap 01 and/or hap 03 and/or hap 07 and/or hap 10 and/or 7 - 12 hap 01 and/or *CETP* 1 - 7 hap 02 (9.9% HDL lowering effect, p=0.006) and/or hap 21 (30.7% Increase HDL, p=0.008) and/or 9 - 12 and/or hap 01 and/or hap 07 and/or LCAT hap 01 and/or hap 03 hap 03 LIPC 1 - 3 hap 01 and/or hap 05 and/or LIPC 4 - 9 hap 01 and/or hap 08 and/or LPL 1 - 6 hap 01 and/or hap 04 and/or LPL 8 - 12 hap 01 and/or hap 11 and/or LDLR hap 04 and/or hap 13 and/or APOC2 hap 05 and/or hap 07 and/or APOA5/APOA4/APOC3/APOA1 gene cluster 1 - 4, 6 hap 01 (52.0% LDL lowering effect, p=0.005) and/or hap 02 and/or 7 - 9 hap 04 and/or 10 - 14 hap 01 and/or hap 11 and/or 15 - 19 hap 01 and/or hap 06 or combinations thereof.

Preferred is a method according to the present invention, wherein a) said at least three SNPs are detected by amplifying all or part of at least one of a APOB, APOE, ABCA1, CETP, LCAT, LIPC, LPL, LDLR, APOC2, APOA5, APOA4, APOC3, and/or APOA1 nucleic acid in said sample using a set of specific primers to produce amplified APOB, APOE, ABCA1, CETP, LCAT, LIPC, LPL, LDLR, APOC2, APOA5, APOA4, APOC3, and/or APOA1 nucleic acids, b) sequencing, e.g. mini-sequencing, the amplified nucleic acids and c) detecting the presence of the at least three SNPs and thereby the presence of said nucleotide alterations in said sample. Preferably, the primers can further contain a detectable label, e.g. a radionuclide, fluorophore, peptide, enzyme, antigen, antibody, vitamin or steroid.

According to another aspect of the present invention, a combination of SNP alleles is detected, consisting of between 3 to 12 SNP alleles per locus (in one gene). This combination allows for an additional improvement in accuracy during the diagnosis.

Most preferably, the combination of haplotypes provides for a detection with a statistical significance of an asymptotic P value < 0.05, preferably P < 0.005 with respect to the diagnosis of a CVD-relevant parameter, such as the phenotypes of LDL, HDL and LDL/HDL ratio and/or contributions of individual haplotypes to these a CVD-relevant parameter(s). In addition, a combination of the haplotypes according to the present invention together with other statistically significant haplotypes can be analysed. Such haplotypes can be easily taken from the respective literature and included in the present set.

In another aspect according to the present invention, a method for APOB, APOE, ABCA1, CETP, LCAT, LIPC, LPL, LDLR, APOC2, APOA5, APOA4, APOC3 and/or APOA1 allele-specific screening for candidate molecules is provided that comprises a) contacting APOB, APOE, ABCA1, CETP, LCAT, LIPC, LPL, LDLR, APOC2, APOA5, APOA4, APOC3 and/or APOA1 with potentially interacting candidate molecules; b) identifying gene-interacting molecules; c) diagnosing the gene allele-specific haplotype based on the method according to the present invention; and d) selecting gene allele-specific interacting molecules based on their different interaction(s) with different gene haplotypes. The invention therefore provides methods for the generation of medicaments and compounds that can specifically reduce the risks of arteriosclerosis involving conditions that is measured using the combination of the SNPs. The compounds thus identified can be used in a method for the production of a APOB, APOE, ABCA1, CETP, LCAT, LIPC, LPL, LDLR, APOC2, APOA5, APOA4, APOC3 and/or APOA1-allele specific pharmaceutical composition, comprising a) the method mentioned above; and b) admixing the identified candidate molecule with a pharmaceutically acceptable carrier or diluent.

Another aspect of the present invention is directed to a method of treatment or prophylaxis in a mammal, in particular a human, that has a genetic predisposition or susceptibility for a cardiovascular disease, condition, or disorder, said method comprising performing the method according to the present invention, and administering to said mammal an effective dose of a therapeutic composition suitable to delay, reduce or prevent said cardiovascular disease, condition, or disorder in said mammal. Preferably, said therapeutic composition comprises a compound selected from the group consisting of cardiovascular disease-effective known drugs or substances, such as statines, fibrates, such as Zocor (simvastatin), Lipitor (atorvastatin), Pravachol (pravastatin), Mevacor (lovastatin), Lescol (fluvastatin), Lipanthyl (fenofibrate), Cedur (bezafibrate), Lipanor (ciprofibrate), Cholestyramine, Colestipol, and fish oils rich in omega-3 triglycerides.

Finally, the invention is also directed to a diagnostic kit and/or a research kit that comprises at least one probe for detecting at least three SNPs in at least on of the loci APOB, APOE, ABCA1, CETP, LCAT, LIPC, LPL, LDLR, APOC2, APOA5, APOA4, APOC3, and/or APOA1 according to the present invention. The kit can contain other compounds such as enzymes, buffers, and/or dyes for performing the method(s) of the present invention. In another example, the kit according to the invention is suitable to perform a chip-based analysis in at least three SNPs in at least one, up to each, of the genes APOB, APOE, ABCA1, CETP, LCAT, LIPC, LPL, LDLR, APOC2, APOA5, APOA4, APOC3, and APOA1. The kit can also include instructions for performing the SNP-analysis and/or the software for a statistical analysis as described herein.

The important finding in the present invention was that the present inventors were able to explain a major part of the genetic variance for LDL, HDL, and especially LDL/HDL ratio in the general German population on the basis of the genetic variants identified in 13 candidate genes important to lipid metabolism. The normal population is highly relevant in terms of investigation since more than half of "normal" people can expect to die of cardiovascular disease. The levels of LDL, HDL and LDL/HDL ratio are important clinical indicators of the cardiovascular risk or degree of protection for each individual. Under controlled standardised physiological conditions, these levels do not vary much in any given individual. The variation between individuals is larger. The variation between members of an out-bred human population is heritable to a degree of about 50% (Higgins, M. (2000). Epidemiology and prevention of coronary heart disease in families. *Am. J Med*., **108,** 387-395, Williams R. R., Hunt S. C., Hopkins P. N., Hasstedt S. J., Berry T. D., Barlow G. K., Stults B. M., Schumacher M. C., Ludwig E. H., et al. (1993) Genetic basis of familial dyslipidemia and hypertension: 15-year results from Utah. *Am. J Hypertens.* **6,** 319S-327S, Jeffreys A. J., Ritchie A, Neumann R. (2000) High resolution analysis of haplotype diversity and meiotic crossover in the human TAP2 recombination hotspot. *Hum. Mol. Genet.* **22**, 725-733).

The present inventors aimed to determine whether or not there is an association of phenotypic variation with genotypic variation in a large number of candidate genes in individuals without signs of lipid disturbances. The genotype-phenotype relationship was established by genotyping "common" SNPs (>3%) in 13 genes (so-called "candidate genes"). About one third was located in intron sequences. However, these SNPs can serve as markers for functionally relevant genetic variants due to high LD between SNPs within the candidate genes. This finding is demonstrated by the fact that a few dominant haplotypes accounted for most of the genotypic variation. The occurrence of LD blocks is in accordance with recent investigations (Daley M. J., Rioux J. D., Schaffner S, Hudson T. J., Lander E. S. (2001) High-resolution haplotype structure in the human genome. *Nat. Genet.* **29:** 229-232; Patil N, Berno A. J., Hinds D. A., Barren W. A., Doshi J. M., Hacker C. R., Kautzer C. R., Lee D. H., Marjoribanks C, McDonough D. P., et al. (2001) Blocks of limited haplotype diversity revealed by high-resolution scanning of human chromosome 21. *Science* **294,** 1719-1723; Reich D. E., Cargill M, Bolk S, Ireland J, Sabeti P. C., Richter D. J., Lavery T, Kouyoumjian R, Farhadian S. F., Ward R. et al. (2001) Linkage disequilibrium in the human genome. *Nature,* **411,** 199-204; Abecasis G. R., Noguchi E, Heinzmann A, Traherne J. A., Bhattacharyya S, Leaves N. I., Anderson G. G., Zhang Y, Lench N. J., Carey A, et al. (2001) Extent and distribution of linkage disequilibrium in three genomic regions. *Am. J Hum. Genet,* **68:** 191-197; Jeffreys A. J., Ritchie A, Neumann R. (2000) High resolution analysis of haplotype diversity and meiotic crossover in the human TAP2 recombination hotspot. *Hum. Mol. Genet.* **22,** 725-733; Gabriel S. B., Schaffner S, Nguyen H, Moore J. M., Roy J, Blumenstiel B, Higgins J, Defeleice M, Lochner A, Faggart M, et al. (2002) The structure of haplotype blocks in the human genome. *Science* **296,** 2225-2229; Phillips M. W. S., Lawrence R, Sachidanandam R, Cardon L. R. et al. (2003) Chromosome-wide distribution of haplotype blocks and the role of recombination hot spots. Nat. Genet. 33, 382-387).

The heritability of lipid phenotypes was lower than those estimated in previous studies (Higgins, M. (2000). Epidemiology and prevention of coronary heart disease in families. *Am. J*. *Med.,* **108,** 387-395; Williams R. R., Hunt S. C., Hopkins P. N., Hasstedt S. J., Berry T. D., Barlow G. K., Stults B. M., Schumacher M. C., Ludwig E. H., et al. (1993) Genetic basis of familial dyslipidemia and hypertension: 15-year results from Utah. *Am. J Hypertens.* **6,** 319S-327S, Knoblauch, H., Busjahn, A., Munter, S., Nagy, Z., Faulhaber, H. D., Schuster, H., Luft, F. C. (1997) Heritability analysis of lipids and three gene loci in twins link the macrophage scavenger receptor to HDL cholesterol concentrations. *Arterioscler. Thromb. Vasc. Biol.,* **17**, 2054-2060; Marenberg, M. E., Risch, N., Berkman, L. F., Floderus, B., de Faire, U. (1994) Genetic susceptibility to death from coronary heart disease in a study of twins. *N. Engl. J. Med.,* **330,** 1041-1046). This result may be due to the greater variation in the non-genetic component in the present sample. Heritability is a relative value that depends on environmental and genetic factors. The families sampled for this analysis were recruited from across Germany and most lipid measurements were performed in routine clinical laboratories. The present inventors certainly do not believe that the present population structure differs from the populations described in earlier studies.

The present inventors found that the association was significant between lipid phenotypes and SNPs as well as haplotypes and/or combinations thereof. The haplotype association was stronger for HDL, while the phase-independent SNP association was stronger for LDL and TC. The interpretation of such differences depends on whether SNPs or haplotypes are functionally relevant or whether they are markers of functional alleles. In most cases common SNP variants are only functionally relevant if they are exonic non-synonymous SNPs or are located in the regulatory elements of genes, for example in promoters or in splice sites. In contrast synonymous or intronic variants may or may not be markers of functionally relevant variants. In principle, it is not possible to predict *a priori,* if a given SNP is functional or simply a marker. Furthermore, it remains unknown if synonymous or intron SNPs are functional or not. For this analysis the present inventors tried to cover the whole genomic sequence of each gene with frequent SNPs. In this scenario, the population history is of importance. The present invention demonstrates, that a major part of the variation of LDL, HDL and LDL/HDL ratio may be explained by variation of common SNPs or SNP-derived haplotypes. These results support the "common trait - common variant" hypothesis for an important metabolic trait (Chakravarty A (1999) Population genetics - making sense out of sequence. *Nat. Genet.,* **22**, 56-60). Templeton (Templeton A. R. (2000) Epistasis and complex traits, pp.41-57 in: Epistasis and the Evolutionary Process, Wolf JB, Brodie IED, Wade MJ editors. Oxford University Press, Oxford) discussed the possibility of epistatic effects in complex traits, exemplified by lipid metabolism. The present inventors explored possible gene-gene-interaction models by regression models and could not find considerable gene-gene interaction effects between different alleles from the same or different genes in the present dataset. Presumably, such effects become distinct only when there is a significant deviation from steady-state physiology. In addition, genes not included in this analysis might account for the remaining polygenic background. These genes, nevertheless, could be included in the analysis of the present invention.

The extended analysis leads to some changes in the conclusions from the present earlier paper, while other results were confirmed and solidified (Knoblauch H, Bauerfeind A, Krahenbuhl C, Daury A, Rohde K, Bejanin S, Essioux L, Schuster H, Luft F. C, Reich JG. (2002) Common haplotypes in five genes influence genetic variance of LDL and HDL cholesterol in the general population. *Hum. Mol. Genet.* **11,** 1477-1485). In general, neither the genetic variance nor the environmental variance changed substantially across both studies. Therefore the present inventors conclude that the increase in the genetic variance explained is due to the additional candidate genes included in this analysis. In the present previous manuscript the present inventors stated, that: "Haplotypes resulted in greater significance than did single SNPs in the genotype-phenotype association analysis". In this analysis, this pattern is no longer generally applicable. The effects of APOE were confirmed for TC, LDL and LDL/HDL ratio, but nor for HDL and TGL. CETP effects were consistent. In addition CETP SNPs effect TC and CETP haplotypes effect TC, TGL and LDL. LCAT did not show any association in either data set. The weak LPL effect on HDL could not be confirmed. The effect of LDLR SNPs was confirmed for the LDL/HDL ratio. In addition the present inventors now found a SNP effect on LDL and HDL, while the LDLR haplotype effect was not confirmed. The present inventors did not find a SNP effect of *LIPC* on the LDL/HDL ratio. The *LIPC* haplotype effect on LDL and the LDL/HDL ratio was consistent and additionally shown for TGL. The differences in significance levels are most likely due to a greater statistical power in the new data set. The linkage disequilibrium (LD) pattern for the additional genes is identical to the LD pattern described earlier. Between 3 and 5 frequent haplotypes accounted for about 80% of the whole sample.

New in the present invention is an analysis of the quantitative contributions of individual genes on the phenotypes LDL, HDL and LDL/HDL ratio. The present inventors were not surprised to find a large contribution of *APOE* to LDL and LDL/HDL ratio. This gene served as the present positive control. Sing and Davignon (Sing C. F. & Davignon J. (1985) The role of apolipoprotein E polymorphism in determining normal plasma lipid and lipoprotein variation.

*Am. J. Hum. Genet.* 37,268-285) reported that *APOE* accounted for about 10% of the LDL phenotypic variance, which is very much in accord with the present findings (polygenic variance of LDL is 26%; 67% of this variance is due to genes analysed; 50% are contributed by APOE). *CETP,* which functions as a junction between LDL and HDL metabolism, had a substantial influence on both these phenotypes. *ABCA1* plays a pivotal role in reverse cholesterol transport and indeed contributed considerably to the HDL phenotype.

Morabia et al. (Morabia, A., Cayanis, E., Costanza, M. C., Ross, B. M., Flherty, M. S., Alvin, G. B., Das, K., Conrad Gilliam, T. (2003) Association of extreme blood lipid profile phenotypic variation with 11 reverse cholesterol transport genes and 10 non-genetic cardiovascular disease risk factors. *Hum. Mol. Genet.* **12**, 2733-2743) recently reported on extreme blood-lipid profiles in a cohort of patients who underwent genotyping in 11 lipid metabolism-relevant genes. They relied on a population-based sample and had at their disposal numerous demographic risk parameters. In their association study, they classified 185 subjects as "cases" who were in the upper third of LDL and lower third of HDL values (worst-possible LDL/HDL ratio). They tested numerous SNPs and genes that were also examined in the present invention. The entire sample comprised 1708 persons. They then performed a logistic regression analysis and showed that a subset of 9 genetic variants in 7 candidate genes, coupled with 5 environmental risk factors, produced a model that may be relevant for future risk assessment. Genes that were in common with the present invention included the *ABCA1, APOA5lA4*/*C3*/*A1* gene cluster, *APOE, CETP, LPL, LIPC,* and *LCAT* genes.

The present inventors believe that important perspectives can arise from the present invention. The present notion that the haplotypes the present inventors describe have prognostic significance for further confirmation will need further testing. If the present haplotypes in combination can indeed predict the occurrence of heart attack or stroke, the reliable use of SNPs to predict human disease would be realised.

In yet another surprising embodiment of the present invention, the inventors particular haplotypes having significant influence on the quantitative phenotype as follows.

**Table 6**

| **gene** | **SNP no. see table 1** | **haplotype** | **SNP code** | **nucleotides** | **frequency** | **effect** |
|---|---|---|---|---|---|---|
| *APOB* | 1 - 4 | hap 07 | 2211 | A..T..C..T | 28.0% | **Increase LDL** (p=0.002) |
| | 5 - 9 | hap 05 | 22112 | T..C..C..C..T | 19.8% | **LDL lowering effect** (p=0.002) |
| *CETP* | 1 - 7 | hap 02 | 1111112 | C..G..G..C..C..C..A | 9.9% | **HDL lowering effect** (p=0.006) |
| | 1-7 | hap 21 | 2122222 | T..G..T..T..T..A..A | 30.7% | **Increase HDL** (p=0.008) |
| *APOA5*/*APOA4*/ *APOC3*/ *APOA1 gene cluster* | 1 - 4, 6 | hap 01 | 11111 | A..G..C..T..T | 52.0% | **LDL lowering effect** (p=0.005) |

p indicates the significance of the median influence (average effect) of the respective haplotypes on the quantitative phenotype. The values stem from the linear regression and are based on the T-test.

The present invention shall now be further described in the following examples with respect to the accompanying drawings without being limited thereto, wherein

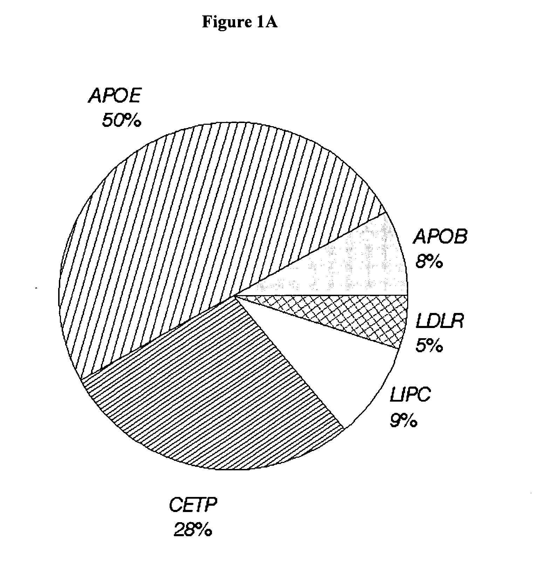

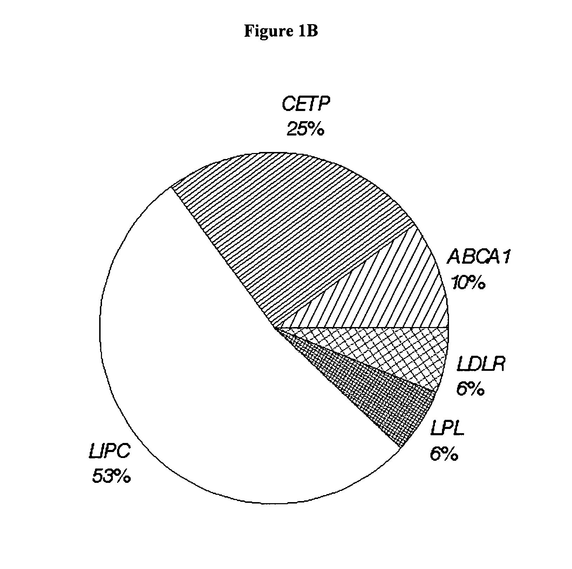

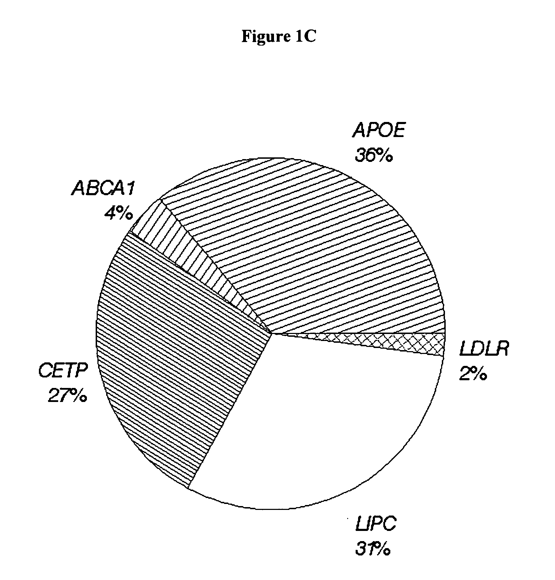

Figure 1 shows a pie diagram that gives the relative contributions of haplotypes in genes contributing >0% to the genetic variance on the phenotypes LDL (figure 1A), HDL (figure 1B), and LDL/HDL ratio (figure 1 C), even if not statistically significant. The LDL/HDL ratio is a clinically highly relevant expression. These data show the relative contributions of the various genes. Genes with a contribution of 0% were excluded. *APOE* and *CETP* mainly influenced the variation in LDL. For HDL, the *LIPC* and *CETP* genes contributed the most. For the LDL/HDL ratio, the major contributors were *APOE, CETP, and LIPC.*

### Examples

### Methods

### Study population

The present inventors selected 218 extended families from the present genetic field working program according to methods outlined elsewhere (Schuster, H., Lamprecht, A., Junghans, C., Dietz, B., Baron, H., Nothnagel, M., Muller-Myhsok, B., Luft, F. C. (1998) Approaches to the genetics of cardiovascular disease through genetic fieldwork. *Kidney. Int.,* **53**, 1449-1454). All subjects completed a medical questionnaire and were examined by their family physician or by the present inventors. The present university's committee on human subjects approved the protocol and written informed consent was obtained from all participants. Families with familial hypercholesterolemia, familial-combined hyperlipidemia, or familial hypertriglyceridemia were excluded, as were families with patients who had known secondary causes of hyperlipidemia. The present inventors focused instead on families without known primary or secondary lipid disturbances. The persons in this analysis were either related to someone with heart disease or were recruited via the family of the spouses of a patient with heart disease. However, none had symptoms of angina pectoris or had known heart disease. The extended families were split into 250 nuclear families that contained at least two adult children. A total of 1054 subjects were included in the study.

### Measurement of lipid traits and genotyping

SNPs were selected from the literature and from public databases (http://www.ncbi.nlm.nih.gov/SNP/). SNP genotyping was performed by DNA sequencing and pyrosequencing (Ronaghi M. (2003) Pyrosequencing for SNP genotyping. *Methods Mol. Biol.* **212,** 189-195). TC, HDL, and TGL were measured in serum by automated methods and LDL was calculated by the Friedewald formula. Further analysis required a Gaussian normal distribution of phenotypes. Therefore, the present inventors took the logarithms of the original lipid measurements and transformed these values to a sample mean of zero and a sample variance of unity. The logarithmic transformation resulted in a fairly satisfactory Gaussian distribution. No correction for age and gender was done to avoid loss of age and gender-dependent genetic effects on the phenotypic variance. The present inventors also inspected the demographic data and assured ourselves that these results were similar to known population values.

### Statistical genetic analysis

All SNPs were in Hardy-Weinberg equilibrium. The present inventors constructed haplotypes in all nuclear families using an E-M algorithm, which uses the nuclear family information (Rohde, K., and Fuerst, R. (2001) Haplotyping and estimation of haplotype frequencies for closely linked biallelic multilocus genetic phenotypes including nuclear family information. *Hum. Mutat.,* **17**,289-95). Complete haplotypes could be assigned to 625 subjects. The presence or absence of haplotypes was coded as absent (=0), present once (=1) or twice (=2).

### Variance component analysis

To take advantage of the family structures, the present inventors performed a variance component analysis for family data. The analysis uses the QTDT program: http://www.well.ox.ac.uk/asthma/QTDT (Abecasis, G. R., Cookson, W. O. C., and Cardon, L. R. (2000) Pedigree tests of transmission equilibrium. *Eur. J. Hum. Genet.,* **8,** 545-551.; Abecasis, G. R., Cardon, L. R., and Cookson, W. O. (2000) A general test of association for quantitative traits in nuclear families. *Am. J. Hum. Genet.,* **66,** 279-292). First a separation into an environmental (Vₑₙᵥ) and a polygenic variance (V_{poly}) component was performed. This analysis compares variance with covariance weighted by the kinship coefficient between individuals based on phenotype data only (Null Model of table 3).

### Partition ofpolygenic and locus-associated additive variance components

The total variance (=1) was partitioned into Vₑₙᵥ , V_{poly}, and in the full model Vₕₐₚ (variance attributed to haplotypes due to association) or V_{SNP} (variance attributed to SNP variables), respectively The contribution of SNPs or SNP haplotypes may be modelled as fixed effects in a multivariate-normal-distribution genetic model as specified by Fulker et al. (Fulker, D. W., Cherny, S. S., Sham, P. C., and Hewitt, J. K. (1999) Combined linkage and association analysis for quantitative traits. *Am. J. Hum. Genet.* **64,** 259-267), and extended to arbitrary pedigrees by Abecasis et al. (Abecasis, G. R., Cardon, L. R., and Cookson, W. O. (2000) A general test of association for quantitative traits in nuclear families. *Am. J Hum. Genet.,* **66,** 279-292). Here, the association is measured as a regression parameter, whereas the genetic influence at non-measured loci (polygenic variance) is modelled as a residual covariance term proportional to the kinship between two individuals (zero, if unrelated, > 0 in a family). Significance of the variance-component-model after estimating the multiple fixed effects was tested as χ² -test derived from likelihood ratio (LR) (Amos, C. I. (1994) Robust Variance-Components Approach for Assessing Genetic Linkage in Pedigrees. *Am. J. Hum. Genet.* **54,** 535-543; Fulker, D. W., Cherny, S. S., Sham, P. C., and Hewitt, J. K. (1999) Combined linkage and association analysis for quantitative traits. *Am. J. Hum. Genet.* **64**, 259-267; Sham, P. C., Cherny, S. S., Purcell, S. and Hewitt, J. K. (2000) Power of linkage versus association analysis of quantitative traits, by use of variance components models, for sibship data. *Am. J. Hum. Genet.,* **66,** 1616-1630; Abecasis, G. R., Cookson, W. O. C., and Cardon, L. R. (2000) Pedigree tests of transmission equilibrium. *Eur. J. Hum. Genet.,* **8,** 545-551; and Abecasis, G. R., Cardon, L. R., and Cookson, W. O. (2000) A general test of association for quantitative traits in nuclear families. *Am. J. Hum. Genet.,* **66**, 279-292).

The non-zero covariance necessitated the use of a non-linear maximum-likelihood estimator, although the difference to an independent-error model (least squares estimation) is only slight in the present data. The present inventors found that the number of independent haplotype parameters (193 out of 625 degrees of freedom) was too large to permit the convergence of the ML optimisation procedure of the QTDT software. The present inventors therefore considered only frequent (> 5%) haplotypes as separate independent variables and pooled rare haplotypes into one group. For the calculation of percentages of explained genetic variance the present inventors considered Vₕₐₚ or _{SNP} relative to Vₕₐₚ or _{SNP} + V_{poly} in haplotype- or SNP-based multiple models. The possibility of population admixture was tested by splitting the individual genotype into a between-family and a within-family component (Abecasis, G. R., Cookson, W. O. C., and Cardon, L. R. (2000) Pedigree tests of transmission equilibrium. *Eur. J. Hum. Genet.,* **8**, 545-551; Abecasis, G. R., Cardon, L. R., and Cookson, W. O. (2000) A general test of association for quantitative traits in nuclear families. *Am. J Hum. Genet.,* **66**, 279-292). The present inventors did not find significant indications of admixture in the present ethnically homogenous sample.

### Relative contribution of genes to phenotypic variance

Significance of the variance component attributable to a locus was tested by a likelihood ratio test between the likelihood of the full multiple SNP or haplotype model and the likelihood of the model with exclusion of the locus tested (table 4). The relative contribution attributable to each gene locus was assessed by calculating the extra-variance due to regression on a tested locus, again by including/excluding the respective variables from the multiple model. This value is biased (Boerwinkle E., Sing C. F. (1986) Bias of the contribution of single locus effects to the variance of a quantitative trait. *Am. J. Hum. Genet.,* **39,** 137-144; Draper N. R. and Smith H. (1998) Applied Regression Analysis 3d ed., ch. 10, Wiley-Interscience, New York; Wijsman E. M., Nur N. (2001) On estimating the proportion of variance in a phenotypic trait attributable to a measured locus. *Hum. Hered.,* 51: 145-149). However, the expected value of the bias (estimate of mean residual variance multiplied by the number of parameters at the tested locus) can be deducted from the variance estimate. Resulting negative variance estimates were interpreted to be zero. Estimated relative contributions between 5 - 10 % are not statistically robust. The resulting relative values of the corrected extra-variance in the haplotype model were combined into the pie charts of figure 1.

The present inventors studied 93 SNPs in 13 lipid-regulatory genes. The present inventors present the SNP identification number, position, their allele frequencies, sequence information and pseudonyms in table 1. 21 SNPs had a frequency below 5%, 12 SNPs a frequency between 5 and 10%, 12 SNPs a frequency between 10 and 20% and 48 SNPs a frequency above 20%. About one third was located within exons, one third within introns and the remaining SNPs in 5'- and 3'-regions of the respective genes. In table 2 all haplotypes are shown. Depending upon the size of the gene and the number of SNPs per gene subhaplotypes were build. In table 3, the present inventors provide demographic data concerning the subjects' age, total cholesterol (TC), HDL, LDL, and LDL/HDL ratio. TC and LDL increased with increasing age. Men had higher TC concentrations, higher LDL values, and lower HDL values than women in every generation.

Table 4 provides the results of the variance component QTDT analysis. In the "null model" (column 2 and 3) the phenotype variance was partitioned into an environmental, family-independent (Vₑₙᵥ), and a family-related genetic (V_{poly}) component. Genotype data are not yet included. The present inventors estimated that 21% of the total variance could be ascribed to genetic effects for total cholesterol, 10% for triglycerides, 26% for LDL, 38% for HDL, and 28% for the LDL/HDL ratio.

To test whether or not the genetic component can be attributed to the candidate genes, the present inventors extended the standard genetic model by introducing haplotypes (column 4 - 8) and SNP genotypes (column 9 - 13) as covariates. The result, displayed in table 4, is a further partition of the genetic component into Vₕₐₚ (attributed to the haplotypes) or V_{SNP} (attributed to SNP genotypes) and V_{poly} (polygenic and family-shared background effects, which cannot reliably be distinguished in nuclear families). The non-shared environmental component Vₑₙᵥ (column 5 and 10) remained approximately at its previous value. The significance of introducing SNP and haplotype effects was tested using a likelihood ratio-test (LR-test) derived from the likelihoods of the "null model" (without genotypes) and the "full model" (genotypes included). The partition was highly significant for LDL, HDL and LDL/HDL-ratio and not significant for triglyerides. For total cholesterol SNPs gave significant result, while haplotypes did not (column 7 and 12). The interpretation must consider that total cholesterol is a mixture of discordant fractions and that triglycerides vary widely and are difficult to standardise. This state-of-affairs tends to increase the "environmental" variance component relative to the genetic component.

The haplotype variation accounted for the major part of the genetic variance for LDL, HDL and LDL/HDL ratio. For LDL, the association with haplotypes explained 67%, for HDL 58%, and for the LDL/HDL ratio 99% of the genetic variance (column 8). Modelling of SNPs as independent variables also explained a major part of the genetic variance, except in the case of HDL where there seems to be a substantial fraction not accounted for by the measured loci (column 13). Haplotypes improved the explanation of HDL variance compared to SNPs. For LDL SNPs were the better predictors. In the case of the LDL/HDL both models gave equal results.

Table 5 shows association effects of single genes by comparing likelihoods excluding and including the SNPs and haplotypes of the respective gene. Panel (a) shows the association of haplotypes with the lipid phenotypes. Only significant gene contributions are shown. For LDL, *APOE* and *CETP* exerted significant effects (p < 0.05). For HDL, *LIPC* and *CETP* haplotypes contribute significantly. Haplotypes of *APOE, CETP,* and *LIPC* significantly predicted the LDL/HDL ratio. Panel (b) shows the association with single SNPs. Again the effect of *APOE* on TC, LDL and LDL/HDL is visible.

Single nucleotide polymorphisms (SNPs) and derived haplotypes within multiple genes may explain genetic variance in complex traits; however, this hypothesis has not been rigorously tested. In an earlier study the present inventors analysed 6 genes and have now expanded this investigation to include 13 genes. The present inventors studied 250 families including 1054 individuals and measured lipid phenotypes. The present inventors focused on low-density cholesterol (LDL) high-density cholesterol (HDL), and their ratio (LDL/HDL). A component analysis of the phenotypic variance relying on a standard genetic model showed that the genetic variance on LDL explained 26%, on HDL explained 38%, and on LDL/HDL explained 28% of the total variance, respectively. Genotyping of 93 SNPs in 13 lipid-relevant genes generated 230 haplotypes. The association of haplotypes in all the genes tested explained a major fraction of the genetic phenotypic variance component. For LDL, the association with haplotypes explained 67% and for HDL 58% of the genetic variance relative to the polygenic background. The present inventors conclude that these haplotypes explain most of the genetic variance in LDL, HDL, and LDL/HDL in these representative German families. An analysis of the contribution to the genetic variance at each locus showed that *APOE* (50%), *CETP* (28%), LIPC (9%), APOB (8%), and LDLR (5%) influenced variation in LDL. LIPC (53%), *CETP* (25%), *ABCA1* (10%), *LPL* (6%), and *LDLR* (6%) influenced the HDL variance. The LDL/HDL ratio was primarily influenced by *APOE* (36%), *CETP* (27%), *LIPC* (31%). This expanded analysis substantially increases the explanation of genetic variance on these complex traits.

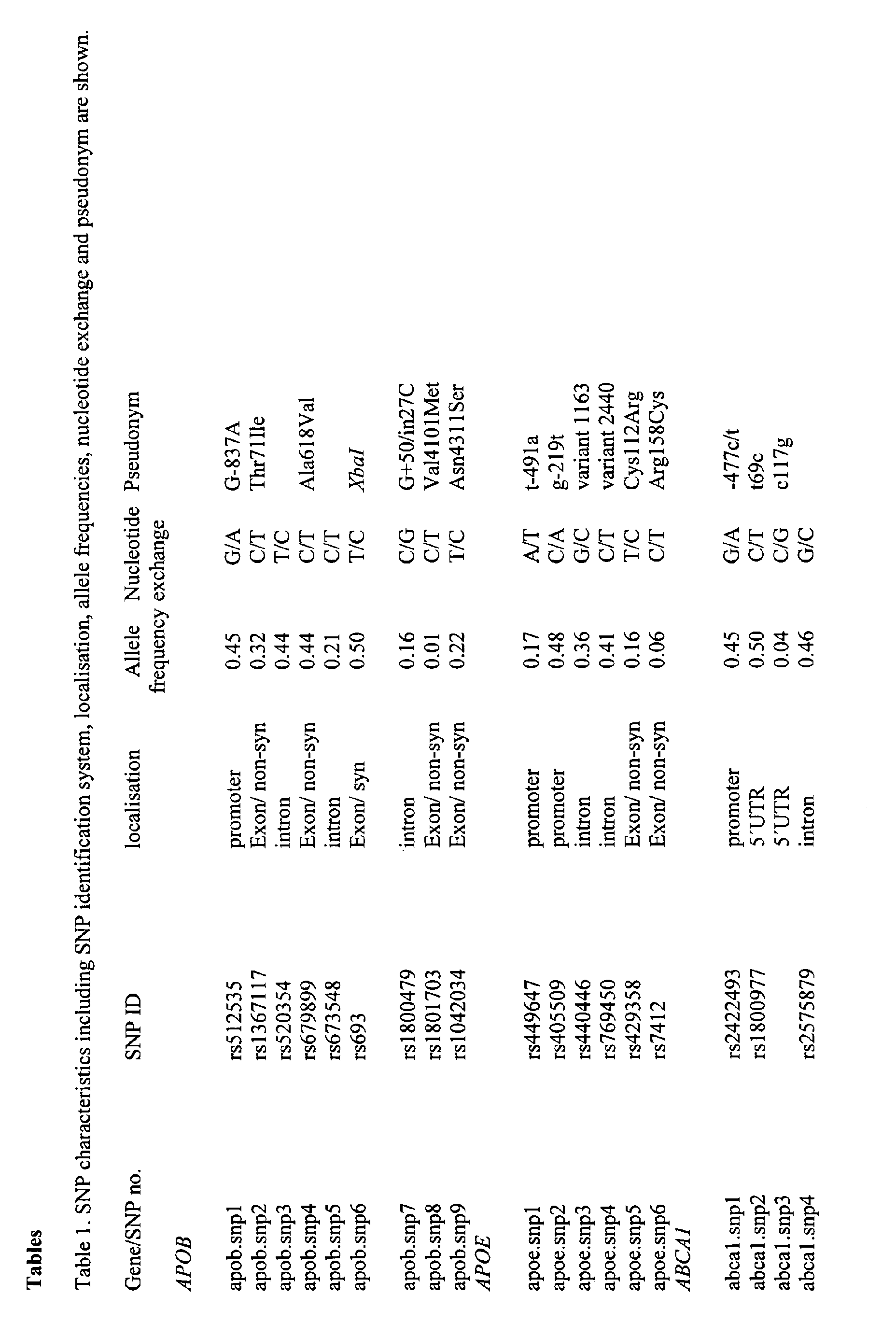

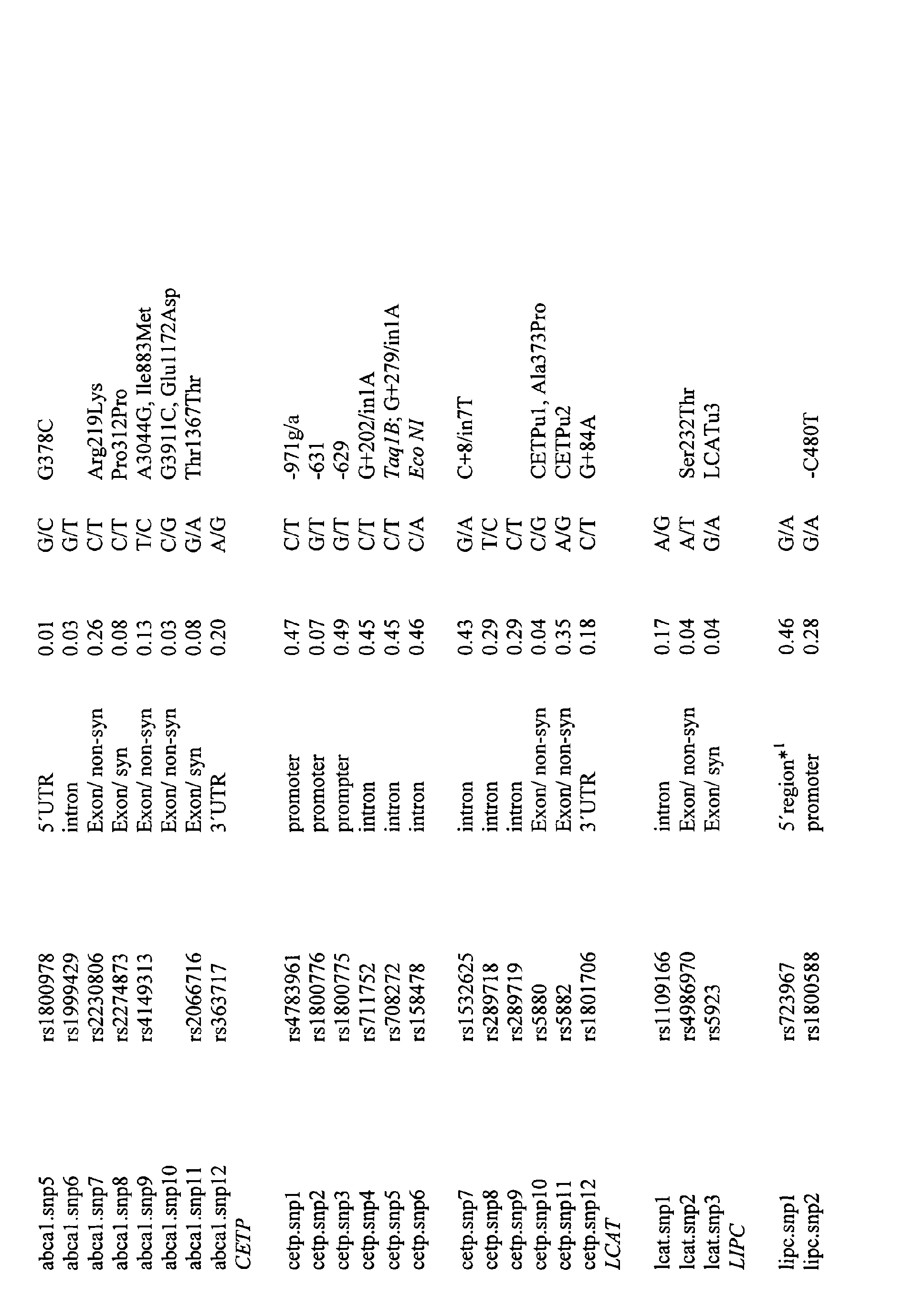

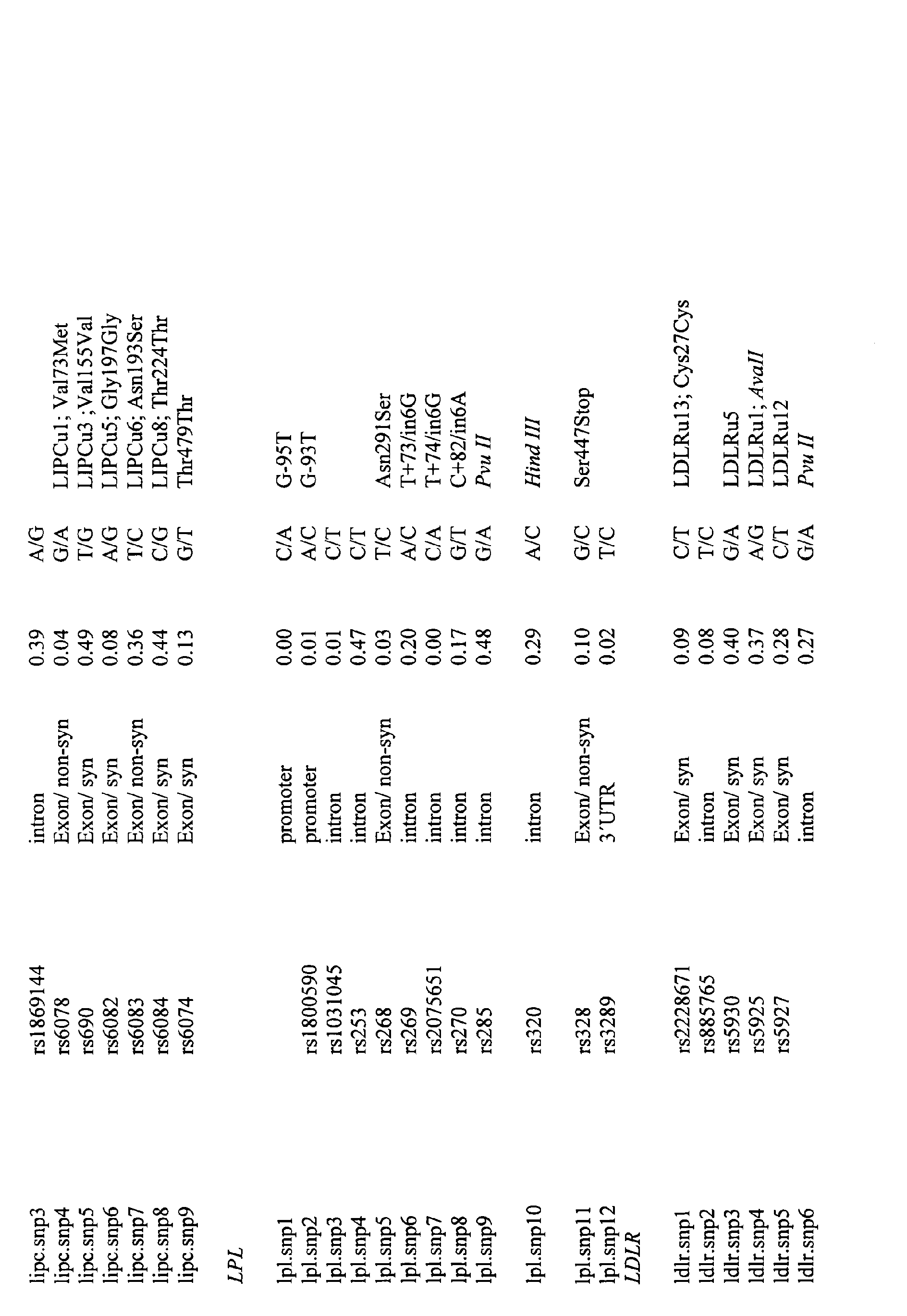

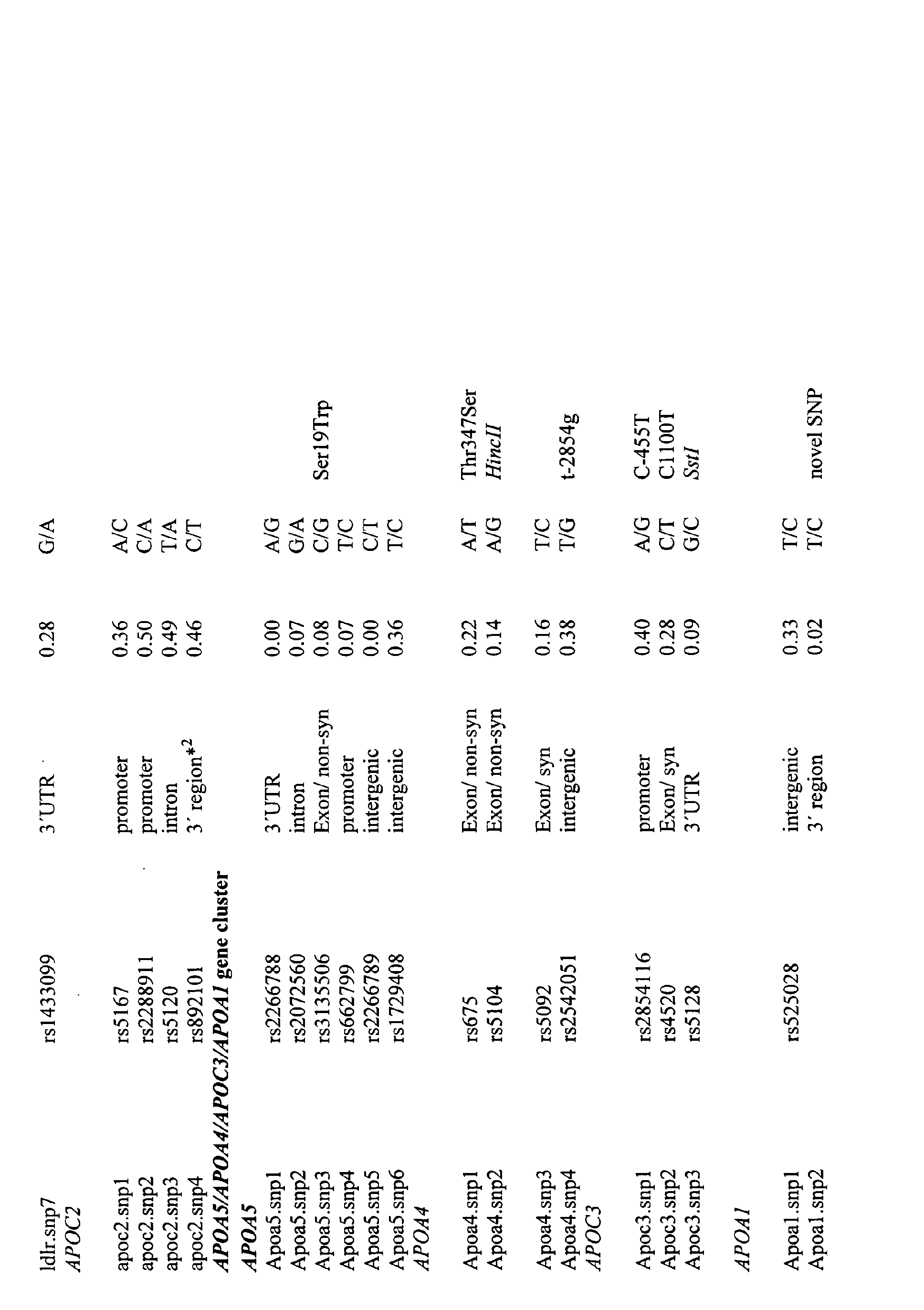

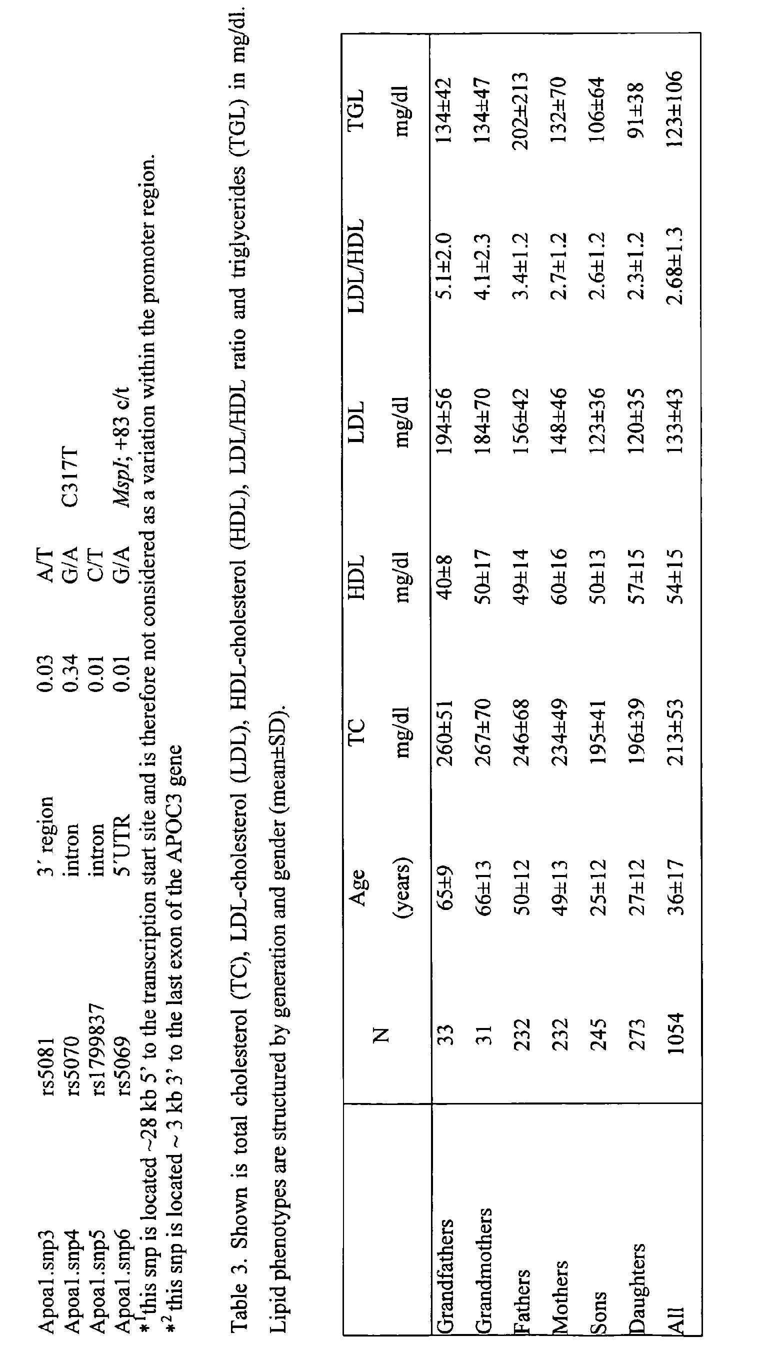

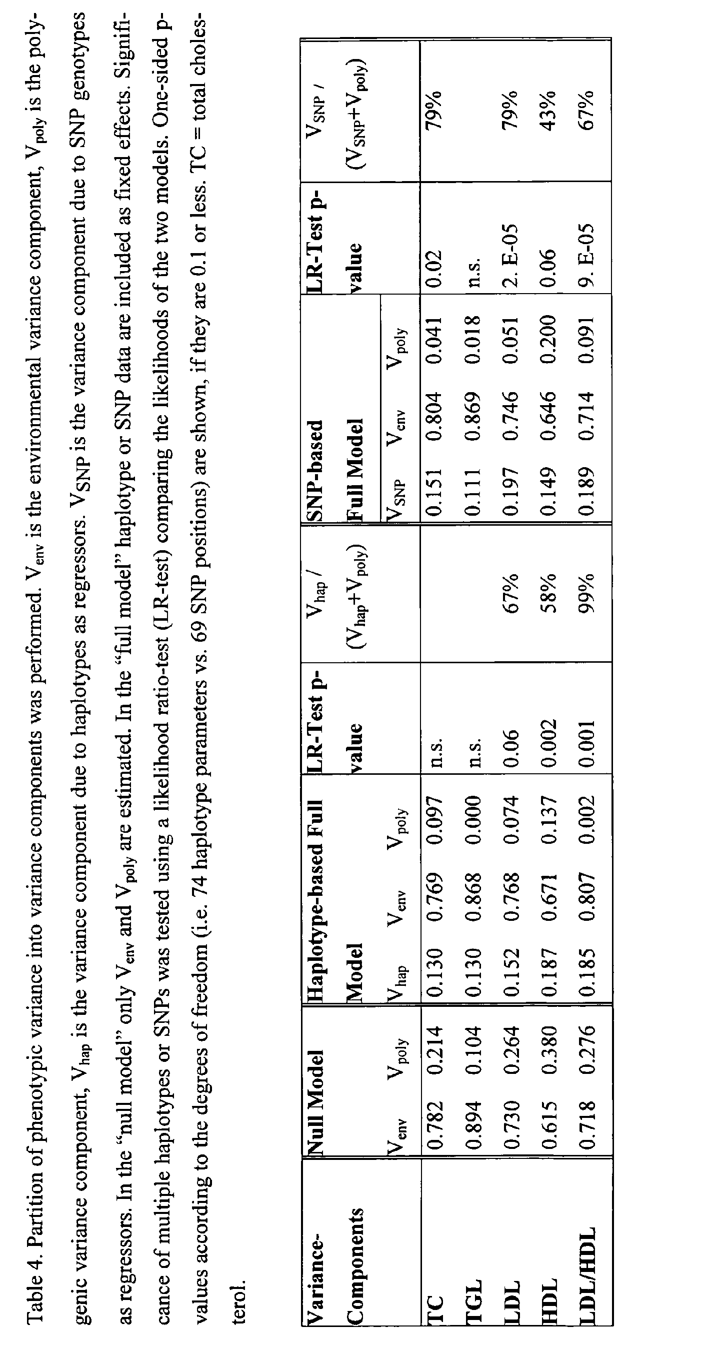

Table 2: Haplotypes, were estimated based on unrelated founder individuals using an EM-algorithm. Depending upon the size of the gene, haploytpes were split into subhaplotypes. SNP no. refers to the SNP numbering system as given in table 1. SNPs are coded as 1 or 2 (1 = frequent allele, 2 = minor allele). Nucleotide sequence and haplotype frequency are shown.

| **gene** | **SNP no.** | **haplotype** | **SNP code** | **nucleotides** | **frequency** |
|---|---|---|---|---|---|
| *APOB* | | | | | |
| | 1 - 4 | hap 01 | 1111 | G..C..T..C | 15.8% |
| | | hap 02 | 1121 | G..C..C..C | 0.3% |
| | | hap 03 | 1122 | G..C..C..T | 42.8% |
| | | hap 04 | 2111 | A..C..T..C | 7.3% |
| | | hap 05 | 2112 | A..C..T..T | 0.1% |
| | | hap 06 | 2122 | A..C..C..T | 5.2% |
| | | hap 07 | 2211 | A..T..C..T | 28.0% |
| | | hap 08 | 2222 | A..T..C..T | 0.5% |

| *APOB* | | | | | |
|---|---|---|---|---|---|
| | 5 - 9 | hap 01 | 11111 | C..T..C..C..T | 52.1% |
| | | hap 02 | 12111 | C..C..C..C..T | 10.8% |
| | | hap 03 | 12112 | C..C..C..C..C | 0.3% |
| | | hap 04 | 12211 | C..C..G..C..T | 16.3% |
| | | hap 05 | 22112 | T..C..C..C..T | 19.8% |
| | | hap 06 | 22122 | T..C..C..T..C | 0.6% |
| | | hap 07 | 22212 | T..C..G..C..C | 0.1% |

| *APOE* | | | | | |
|---|---|---|---|---|---|
| | 1 - 6 | hap 01 | 111111 | A..C..G..C..T..C | 0.7% |
| | | hap 02 | 111112 | A..C..G..C..T..T | 4.5% |
| | | hap 03 | 111121 | A..C..G..C..C..T | 2.6% |
| | | hap 04 | 111211 | A..C..G..T..T..C | 40.7% |
| | | hap 05 | 112111 | A..C..C..C..T..C | 0.2% |
| | | hap 06 | 121111 | A..A..G..C..T..C | 0.3% |
| | | hap 07 | 121121 | A..A..G..C..C..C | 10.6% |
| | | hap 08 | 121211 | A..A..G..T..T..C | 0.1% |
| | | hap 09 | 122111 | A..A..C..C..T..C | 23.4% |
| | | hap 10 | 122112 | A..A..C..C..T..T | 0.2% |
| | | hap 11 | 122121 | A..A..C..C..C..C | 0.2% |
| | | hap 12 | 211111 | T..C..G..C..T..C | 0.5% |
| | | hap 13 | 211112 | T..C..G..C..T..T | 3.3% |
| | | hap 14 | 211211 | T..C..G..T..T..C | 3.2% |
| | | hap 15 | 211221 | T..C..G..T..C..C | 0.1% |
| | | hap 16 | 221121 | T..A..G..C..C..C | 0.3% |
| | | hap 17 | 222111 | T..A..C..C..T..C | 8.9% |

| *ABCA1* | | | | | |
|---|---|---|---|---|---|
| | 1,3-6 | hap 01 | 11111 | G..C..G..G..G | 22.6% |
| | | hap 02 | 11112 | G..C..G..G..T | 1.2% |
| | | hap 03 | 11211 | G..C..C..G..G | 30.0% |
| | | hap 04 | 11212 | G..C..C..G..T | 0.1% |
| | | hap 05 | 12111 | G..G..G..G..G | 2.0% |
| | | hap 06 | 12211 | G..G..C..G..G | 0.9% |
| | | hap 07 | 21111 | A..C..G..G..G | 22.6% |
| | | hap 08 | 21112 | A..C..G..G..T | 2.4% |
| | | hap 09 | 21121 | A..C..G..C..G | 0.1% |
| | | hap 10 | 21211 | A..C..C..G..G | 18.0% |
| | | hap 11 | 21212 | A..C..C..G..T | 0.1% |

| *ABCA1* | | | | | |
|---|---|---|---|---|---|
| | 7 - 12 | hap 01 | 111111 | C..C..T..C..G..A | 48.0% |
| | | hap 02 | 111112 | C..C..T..C..G..G | 15.2% |
| | | hap 03 | 111121 | C..C..T..C..A..A | 2.4% |
| | | hap 04 | 111122 | C..C..T..C..A..G | 0.3% |
| | | hap 05 | 111211 | C..C..T..G..G..A | 0.4% |
| | | hap 06 | 111212 | C..C..T..G..G..G | 0.4% |
| | | hap 07 | 112111 | C..C..C..C..G..A | 4.4% |
| | | hap 08 | 112121 | C..C..C..C..A..A | 1.5% |
| | | hap 09 | 112211 | C..C..C..G..G..A | 0.2% |
| | | hap 10 | 121111 | C..T..T..C..G..A | 0.7% |
| | | hap 11 | 121211 | C..T..T..G..G..A | 0.9% |
| | | hap 12 | 211111 | T..C..T..C..G..A | 9.8% |
| | | hap 13 | 211112 | T..C..T..C..G..G | 1.7% |
| | | hap 14 | 211121 | T..C..T..C..A..A | 0.4% |
| | | hap 15 | 211122 | T..C..T..C..A..G | 0.3% |
| | | hap 16 | 212111 | T..C..C..C..G..A | 3.3% |
| | | hap 17 | 212112 | T..C..C..C..G..G | 0.4% |
| | | hap 18 | 212121 | T..C..C..C..A..A | 1.7% |
| | | hap 19 | 212211 | T..C..C..G..G..A | 0.7% |
| | | hap 20 | 221111 | T..T..T..C..G..A | 5.6% |
| | | hap 21 | 221112 | T..T..T..C..G..G | 0.7% |
| | | hap 22 | 221121 | T..T..T..C..A..A | 0.4% |
| | | hap 23 | 112112 | C..C..C..C..G..G | 0.1% |
| | | hap 24 | 112212 | C..C..C..G..G..G | 0.1 % |
| | | hap 25 | 211211 | T..C..T..G..G..A | 0.2% |
| | | hap 26 | 212212 | T..C..C..G..G..G | 0.1% |

| *CETP* | | | | | |
|---|---|---|---|---|---|
| | 1 - 7 | hap 01 | 1111111 | C..G..G..C..C..C..G | 23.7% |
| | | hap 02 | 1111112 | C..G..G..C..C..C..A | 9.9% |
| | | hap 03 | 1121111 | C..G..T..C..C..C..G | 0.7% |
| | | hap 04 | 1121112 | C..G..T..C..C..C..A | 0.1% |
| | | hap 05 | 1121122 | C..G..T..C..C..A..A | 0.1% |
| | | hap 06 | 1122212 | C..G..T..T..T..C..A | 0.3% |
| | | hap 07 | 1122221 | C..G..T..T..T..A..G | 8.1% |
| | | hap 08 | 1122222 | C..G..T..T..T..A..A | 0.3% |
| | | hap 09 | 1211111 | C..T..G..C..C..C..G | 6.4% |
| | | hap 10 | 1211112 | C..T..G..C..C..C..A | 0.2% |
| | | hap 11 | 2111111 | T..G..G..C..C..C..G | 2.6% |
| | | hap 12 | 2111112 | T..G..G..C..C..C..A | 6.0% |
| | | hap 13 | 2112212 | T..G..G..T..T..C..A | 0.0% |
| | | hap 14 | 2121111 | T..G..T..C..C..C..G | 4.2% |
| | | hap 15 | 2121112 | T..G..T..C..C..C..A | 0.3% |
| | | hap 16 | 2121121 | T..G..T..C..C..A..G | 0.3% |
| | | hap 17 | 2121122 | T..G..T..C..C..A..A | 0.8% |
| | | hap 18 | 2122211 | T..G..T..T..T..C..G | 0.3% |
| | | hap 19 | 2122212 | T..G..T..T..T..C..A | 0.2% |
| | | hap 20 | 2122221 | T..G..T..T..T..A..G | 4.5% |
| | | hap 21 | 2122222 | T..G..T..T..T..A..A | 30.7% |

| *CETP* | | | | | |
|---|---|---|---|---|---|
| | 9 - 12 | hap 01 | 1111 | C..C..A..C | 66.2% |
| | | hap 02 | 1121 | C..C..G..C | 2.2% |
| | | hap 03 | 1122 | C..C..G..T | 0.1% |
| | | hap 04 | 1211 | C..G..A..C | 3.0% |
| | | hap 05 | 2111 | T..C..A..C | 0.7% |
| | | hap 06 | 2121 | T..C..G..C | 10.6% |
| | | hap 07 | 2122 | T..C..G..T | 17.1% |
| | | hap 08 | 2222 | T..G..G..T | 0.1% |

| *LCAT* | | | | | |
|---|---|---|---|---|---|
| | 1 - 3 | hap 01 | 111 | A..A..G | 80.6% |
| | | hap 02 | 121 | A..T..G | 3.4% |
| | | hap 03 | 211 | G..A..G | 12.6% |
| | | hap 04 | 212 | G..A..A | 3.4% |

| *LIPC* | | | | | |
|---|---|---|---|---|---|
| | 1 - 3 | hap 01 | 111 | G..G..A | 26.0% |
| | | hap 02 | 112 | G..G..G | 17.6% |
| | | hap 03 | 121 | G..A..A | 8.1% |
| | | hap 04 | 122 | G..A..G | 3.8% |
| | | hap 05 | 211 | A..G..A | 20.4% |
| | | hap 06 | 212 | A..G..G | 11.3% |
| | | hap 07 | 221 | A..A..A | 8.9% |
| | | hap 08 | 222 | A..A..G | 3.8% |

| *LIPC* | | | | | |
|---|---|---|---|---|---|
| | 4 - 9 | hap 01 | 111111 | G..T..A..T..C..G | 32.9% |
| | | hap 02 | 111112 | G..T..A..T..C..T | 0.2% |
| | | hap 03 | 111121 | G..T..A..T..G..G | 6.6% |
| | | hap 04 | 111211 | G..T..A..C..C..G | 0.1% |
| | | hap 05 | 111221 | G..T..A..C..G..G | 8.8% |
| | | hap 06 | 111222 | G..T..A..C..G..T | 5.8% |
| | | hap 07 | 112221 | G..T..G..C..G..G | 4.0% |
| | | hap 08 | 121111 | G..G..A..T..C..G | 22.4% |
| | | hap 09 | 121121 | G..G..A..T..G..G | 2.0% |
| | | hap 10 | 121211 | G..G..A..C..C..G | 0.1% |
| | | hap 11 | 121221 | G..G..A..C..G..G | 3.9% |
| | | hap 12 | 121222 | G..G..A..C..G..T | 7.0% |
| | | hap 13 | 122221 | G..G..G..C..G..G | 2.7% |
| | | hap 14 | 122222 | G..G..G..C..G..T | 0.1% |
| | | hap 15 | 211111 | A..T..A..T..C..G | 0.1% |
| | | hap 16 | 221111 | A..G..A..T..C..G | 1.2% |
| | | hap 17 | 221121 | A..G..A..T..G..G | 0.0% |
| | | hap 18 | 221221 | A..G..A..C..G..G | 1.1% |
| | | hap 19 | 221222 | A..G..A..C..G..T | 0.1% |
| | | hap 20 | 222221 | A..G..G..C..G..G | 0.7% |

| *LPL* | | | | | |
|---|---|---|---|---|---|
| | 1 - 6 | hap 01 | 111111 | C..A..C..C..T..A | 53.4% |
| | | hap 02 | 111112 | C..A..C..C..T..C | 0.2% |
| | | hap 03 | 111121 | C..A..C..C..C..A | 2.2% |
| | | hap 04 | 111211 | C..A..C..T..T..A | 24.3% |
| | | hap 05 | 111212 | C..A..C..T..T..A | 17.6% |
| | | hap 06 | 112111 | C..A..T..C..T..A | 0.1% |
| | | hap 07 | 121211 | C..C..C..T..T..A | 0.1% |
| | | hap 08 | 121212 | C..C..C..T..T..C | 0.6% |
| | | hap 09 | 122211 | C..C..T..T..T..A | 0.2% |
| | | hap 10 | 122212 | C..C..T..T..T..C | 0.1 % |
| | | hap 11 | 211212 | A..A..C..T..T..C | 0.1% |
| | | hap 12 | 212211 | A..A..T..T..T..A | 1.0% |

| *LPL* | | | | | |
|---|---|---|---|---|---|
| | 8 - 12 | hap 01 | 11111 | G..G..A..G..T | 46.7% |
| | | hap 02 | 11112 | G..G..A..G..C | 0.1% |
| | | hap 03 | 11211 | G..G..C..G..T | 5.8% |
| | | hap 04 | 11221 | G..G..C..C..T | 0.3% |
| | | hap 05 | 12111 | G..A..A..G..T | 9.4%. |
| | | hap 06 | 12112 | G..A..A..G..C | 1.8% |
| | | hap 07 | 12211 | G..A..C..G..T | 11.1% |
| | | hap 08 | 12221 | G..A..C..C..T | 9.1% |
| | | hap 09 | 21111 | T..G..A..G..T | 1.5% |
| | | hap 10 | 21211 | T..G..C..G..T | 0.2% |
| | | hap 11 | 22111 | T..A..A..G..T | 13.8% |
| | | hap 12 | 22112 | T..A..A..G..C | 0.1% |

| *LDLR* | | | | | |
|---|---|---|---|---|---|
| | 1 - 7 | hap 01 | 1111111 | C..T..G..A..C..G..G | 0.7% |
| | | hap 02 | 1111112 | C..T..G..A..C..G..A | 0.3% |
| | | hap 03 | 1111222 | C..T..G..A..T..A..A | 4.8% |
| | | hap 04 | 1112111 | C..T..G..G..C..G..G | 36.6% |
| | | hap 05 | 1112112 | C..T..G..G..C..G..A | 0.1% |
| | | hap 06 | 1112221 | C..T..G..G..T..A..G | 0.1% |
| | | hap 07 | 1112222 | C..T..G..G..T..A..A | 1.0% |
| | | hap 08 | 1121111 | C..T..A..A..C..G..G | 17.6% |
| | | hap 09 | 1121112 | C..T..A..A..C..G..A | 0.6% |
| | | hap 10 | 1121122 | C..T..A..A..C..A..A | 0.2% |
| | | hap 11 | 1121211 | C..T..A..A..T..G..G | 0.1% |
| | | hap 12 | 1121221 | C..T..A..A..T..A..G | 2.7% |
| | | hap 13 | 1121222 | C..T..A..A..T..A..A | 16.7% |
| | | hap 14 | 1122111 | C..T..A..G..C..G..G | 0.9% |
| | | hap 15 | 1211111 | C..C..G..A..C..G..G | 5.6% |
| | | hap 16 | 1211112 | C..C..G..A..C..G..A | 2.1% |
| | | hap 17 | 1211222 | C..C..G..A..T..A..A | 0.2% |
| | | hap 18 | 2111111 | T..T..G..A..C..G..G | 2.7% |
| | | hap 19 | 2111112 | T..T..G..A..C..G..A | 0.1% |
| | | hap 20 | 2112111 | T..T..G..G..C..G..G | 4.3% |
| | | hap 21 | 2112222 | T..T..G..G..T..A..A | 0.1% |
| | | hap 22 | 2121111 | T..T..A..A..C..G..G | 2.3% |

| *APOC2* | | | | | |
|---|---|---|---|---|---|
| | 1 - 4 | hap 01 | 1111 | A..C..T..C | 0.8% |
| | | hap 02 | 1112 | A..C..T..T | 14.1% |
| | | hap 03 | 1211 | A..A..T..C | 0.2% |
| | | hap 04 | 1212 | A..A..T..T | 0.2% |
| | | hap 05 | 1221 | A..A..A..C | 45.9% |
| | | hap 06 | 2111 | C..C..T..C | 3.6% |
| | | hap 07 | 2112 | C..C..T..T | 33.2% |
| | | hap 08 | 2121 | C..C..A..C | 0.1% |
| | | hap 09 | 2122 | C..C..A..T | 0.2% |
| | | hap 10 | 2212 | C..A..T..T | 0.2% |
| | | hap 11 | 2221 | C..A..A..C | 1.4% |

| *APOA5*/*APOA4*/*APOC3*/*APOA1* gene cluster | | | | | |
|---|---|---|---|---|---|
| *Cluster* | | | | | |
| | 1 - 4, 6 | hap 01 | 11111 | A..G..C..T..T | 52.0% |
| | | hap 02 | 11112 | A..G..C..T..C | 33.4% |
| | | hap 03 | 11122 | A..G..C..C..C | 0.6% |
| | | hap 04 | 11211 | A..G..G..T..T | 7.1% |
| | | hap 05 | 11212 | A..G..G..T..C | 0.2% |
| | | hap 06 | 12112 | A..A..C..T..C | 0.1% |
| | | hap 07 | 12121 | A..A..C..C..T | 6.3% |
| | | hap 08 | 12122 | A..A..C..C..C | 0.3% |

| *Cluster* | | | | | |
|---|---|---|---|---|---|
| | 7 - 9 | hap 01 | 111 | A..A..T | 63.1% |
| | | hap 02 | 112 | A..A..C | 1.9% |
| | | hap 03 | 122 | A..G..C | 13.3% |
| | | hap 04 | 211 | T..A..T | 21.5% |
| | | hap 05 | 221 | T..G..T | 0.1% |

| *Cluster* | | | | | |
|---|---|---|---|---|---|
| | 10 - 14 | hap 01 | 11111 | T..A..C..G..T | 44.9% |
| | | hap 02 | 11112 | T..A..C..G..C | 1.9% |
| | | hap 03 | 11211 | T..A..T..G..T | 0.8% |
| | | hap 04 | 11212 | T..A..T..G..C | 12.6% |
| | | hap 05 | 11222 | T..A..T..C..C | 0.1% |
| | | hap 06 | 12111 | T..G..C..G..T | 0.1% |
| | | hap 07 | 12112 | T..G..C..G..C | 2.6% |
| | | hap 08 | 21111 | G..A..C..G..T | 0.2% |
| | | hap 09 | 21212 | G..A..T..G..C | 0.2% |
| | | hap 10 | 21222 | G..A..T..C..C | 0.1% |
| | | hap 11 | 22111 | G..G..C..G..T | 22.2% |
| | | hap 12 | 22112 | G..G..C..G..C | 1.6% |
| | | hap 13 | 22211 | G..G..T..G..T | 0.1% |
| | | hap 14 | 22212 | G..G..T..G..C | 3.5% |
| | | hap 15 | 22222 | G..G..T..C..C | 9.0% |

| *Cluster* | | | | | |
|---|---|---|---|---|---|
| | 15 - 19 | hap 01 | 11111 | T..A..G..C..G | 44.2% |
| | | hap 02 | 11112 | T..A..G..C..A | 13.0% |
| | | hap 03 | 11121 | T..A..G..T..G | 2.7% |
| | | hap 04 | 11211 | T..A..A..C..G | 0.1% |
| | | hap 05 | 11212 | T..A..A..C..A | 0.2% |
| | | hap 06 | 11221 | T..A..A..T..G | 24.2% |
| | | hap 07 | 11222 | T..A..A..T..A | 0.2% |
| | | hap 08 | 12111 | T..T..G..C..G | 0.1% |
| | | hap 09 | 12221 | C..A..G..C..A | 1.7% |
| | | hap 10 | 12222 | T..T..A..T..A | 0.1% |
| | | hap 11 | 21221 | C..A..A..T..G | 0.1% |
| | | hap 12 | 22211 | C..T..A..C..G | 0.1% |
| | | hap 13 | 22212 | C..T..A..C..A | 0.1% |
| | | hap 14 | 22221 | C..T..A..T..G | 0.5% |
| | | hap 15 | 22222 | C..T..A..T..A | 12.6% |

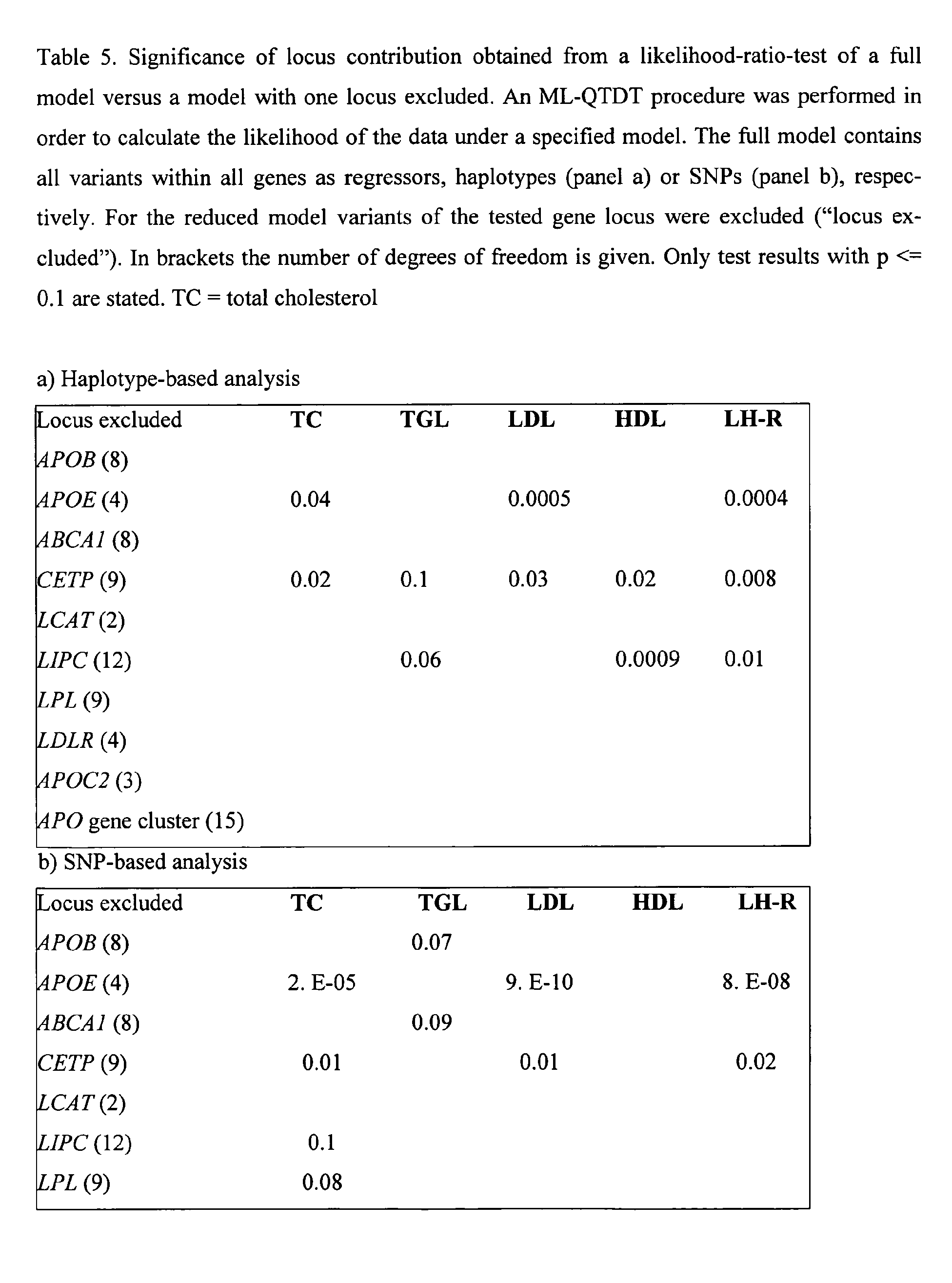

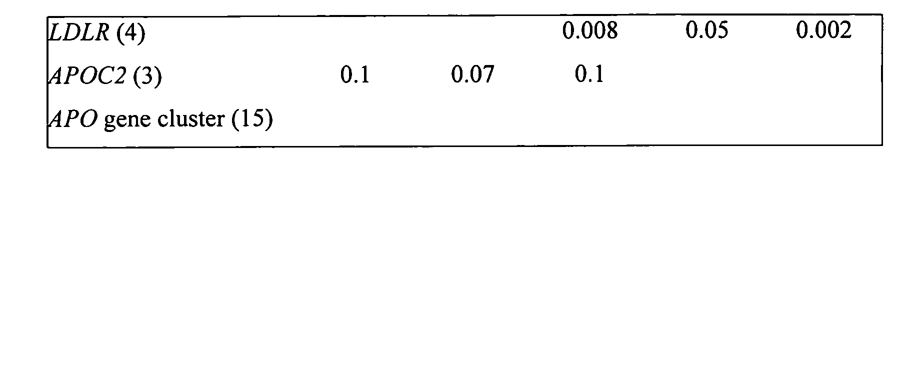

## Claims

1. *An in vitro* method for diagnosing a genetic predisposition or susceptibility for a cardiovascular disease, condition or disorder in a mammal comprising:
detecting in a sample obtained from said mammal at least three single nucleotide polymorphisms (SNPs) in a nucleic acid or fragment thereof wherein said nucleic acid or fragment thereof is derived from at least one of a genomic locus of the group consisting of APOB, APOE, ABCA1, CETP, LCAT, LIPC, LPL, LDLR, APOC2, APOA5, APOA4, APOC3, and APOA1, and
wherein said at least three SNPs are selected from the group consisting of the SNPs rs512535, rs1367117, rs520354, rs679899, rs673548, rs693, rs1800479, rs1801703, and rs1042034 of APOB, rs449647, rs405509, rs440446, rs769450, rs429358, and rs7412 of APOE, rs2422493, rs1800977, rs2575879, rs1800978, rs1999429, rs2230806, rs2274873, rs4149313, rs2066716, and rs363717 of ABCA1, rs4783961, rs1800776, rs1800775, rs711752, rs708272, rs158478, rs1532625, rs289718, rs289719, rs5880, rs5882, and rs1801706 of CETP, rs1109166, rs4986970, and rs5923 of LCAT, rs723967, rs1800588, rs1869144, rs6078, rs690, rs6082, rs6083, rs6084, and rs6074 of LIPC, rs1800590, rs1031045, rs253, rs268, rs269, rs2075651, rs270, rs285, rs320, rs328, and rs3289 of LPL, rs2228671, rs885765, rs5930, rs5925, rs5927, and rs1433099 of LDLR, rs5167, rs2288911, rs5120, and rs892101 of APOC2, rs2266788, rs2072560, rs3135506, rs662799, rs2266789, and rs1729408 of APOA5, rs675, rs5104, rs5092, and rs2542051 of APOA4, rs2854116, rs4520, and rs5128 of APOC3, and rs525028, rs5081rs5070, rs1799837, and rs5069 of APOA1., whereby at least one allele-specific haplotype is determined.

2. Method according to claim 1, wherein the diagnosis identifies individuals that are protected from said cardiovascular disease, condition, or disorder.

3. Method according to claim 1 or 2, wherein the genetic predisposition or susceptibility for a cardiovascular disease, condition, or disorder involves myocardial infarction, cerebrovascular disease, stroke, dementia, thrombosis, pulmonary embolism, renal infarction, and/or abnormal blood lipid levels.

4. Method according to any of claims 1 to 3, wherein said mammal is a human.

5. Method according to any of claims 1 to 4, wherein said nucleic acid is DNA, genomic DNA, RNA, cDNA, hnRNA and/or mRNA.

6. Method according to any of claims 1 to 5, wherein said detection is accomplished by sequencing, mini-sequencing, hybridisation, restriction fragment analysis, oligonucleotide ligation assay or allele specific PCR.

7. Method according to claim 6, wherein
a) said at least three SNPs are detected by amplifying all or part of a nucleic acid of at least one of the loci APOB, APOE, ABCA1, CETP, LCAT, LIPC, LPL, LDLR, APOC2, APOA5, APOA4, APOC3, and APOA1 in said sample using a set of gene specific primers to produce amplified nucleic acid of at least one of the loci APOB, APOE, ABCA1, CETP, LCAT, LIPC, LPL, LDLR, APOC2, APOA5, APOA4, APOC3, and APOA1 nucleic acids,
b) sequencing, the amplified nucleic acid of at least one of the loci APOB, APOE, ABCA1, CETP, LCAT, LIPC, LPL, LDLR, APOC2, APOA5, APOA4, APOC3, and APOA1 nucleic acids, and
c) detecting the presence of the at least three SNPs in at least one of the loci APOB, APOE, ABCA1, CETP, LCAT, LIPC, LPL, LDLR, APOC2, APOA5, APOA4, APOC3, and APOA1, and thereby the presence of said nucleotide alterations in said sample.

8. Method according to any of claims 1 to 7, wherein a combination of haplotypes of the loci APOB, APOE, ABCA1, CETP, LCAT, LIPC, LPL, LDLR, APOC2, APOA5, APOA4, APOC3, and APOA1 is detected, consisting of between 2 to 13 SNP haplotypes.

9. Method according to any of claims 1 to 8, wherein the combination of haplotypes provides for a detection with a statistical significance of an asymptotic P value < 0.05, preferably *P* < 0.005.

10. Method according to any of claims 1 to 9, wherein a combination together with other statistically significant haplotypes is analysed.

11. Method for APOB, APOE, ABCA1, CETP, LCAT, LIPC, LPL, LDLR, APOC2, APOA5, APOA4, APOC3 and/or APOA1 allele-specific screening for candidate molecules, comprising
a) contacting APOB, APOE, ABCA1, CETP, LCAT, LIPC, LPL, LDLR, APOC2, APOA5, APOA4, APOC3 and/or APOA1 with potentially interacting candidate molecules;
b) identifying gene-interacting molecules;
c) diagnosing the allele-specific haplotype based on the method according to any of claims 1 to 9; and
d) selecting allele-specific interacting molecules based on their different interaction(s) with different haplotypes.

12. Method for the production of a APOB, APOE, ABCA1, CETP, LCAT, LIPC, LPL, LDLR, APOC2, APOA5, APOA4, APOC3 and/or APOA1-allele specific pharmaceutical composition, comprising
a) performing a method according to claim 11; and
b) admixing the identified candidate molecule with a pharmaceutically acceptable carrier or diluent.

13. A diagnostic kit and/or a research kit, comprising at least one combination of probes for detecting at least one of the haplotypes according to claim 1.
